# EUROPEAN PATENT SPECIFICATION

(11) **EP 1 409 501 B1**
(45) Date of publication and mention of the grant of the patent: **05.03.2008**
(21) Application number: 01929808.2
(22) Date of filing: 11.05.2001
(51) Int. Cl.: C07H 15/207, A61K 31/70, C07H 3/04, C07H 3/06, C07H 11/04

(54) **INOSITOL PHOSPHOGLYCAN DERIVATIVES AND THEIR MEDICAL USES**
INOSITOLPHOSPHOGLYCANDERIVATE UND IHRE MEDIZINISCHE VERWENDUNG
DERIVES D'INOSITOL PHOSPHOGLYCANE ET UTILISATIONS MEDICALES DE CEUX-CI

(30) Priority: 12.05.2000 GB 0011592; 12.05.2000 US 203599 P; 02.03.2001 US 798124
(43) Date of publication of application: 21.04.2004
(73) Proprietor: Rodaris Pharmaceuticals Limited, Oxford, OX4 4GA (GB)
(72) Inventor: MARTIN-LOMAS, Manuel, 41013 Seville (ES); RADEMACHER, Thomas William, Oxford, Oxfordshire OX1 5EY (GB); CARO, Hugo Noberto, Barcelona 08036 (ES); FRANCOIS, Irene,, Horsell, Woking, Surrey GU21 4BE (GB)
(74) Representative: Kiddle, Simon John
(86) International application number: PCT/GB2001/002083
(87) International publication number: WO 2001/085746

(56) References cited:
- EP-A- 0 845 475
- WO-A-00/39141
- WO-A-98/10791
- WO-A-99/38516
- PATENT ABSTRACTS OF JAPAN vol. 1995, no. 01, 28 February 1995 (1995-02-28) & JP 06 293790 A (KYOWA HAKKO KOGYO CO LTD), 21 October 1994 (1994-10-21)
- MARTIN-LOMAS, M. ET AL.: "Inositolphosphoglycan mediators structurally related to glycosyl phosphatidylInositol anchors: synthesis, structure and biological activity" CHEM. EUR. J., vol. 6, no. 19, pages 3608-21, XP001017985
- MÜLLER, G. ET AL.: "Structure-activity relationship of synthetic phosphoinositolglycans mimicking metabolic insulin action" BIOCHEMISTRY, vol. 37, no. 38, 1998, pages 13421-13436, XP002135715
- MÜLLER, G. ET AL.: "Phophoinositolglycan-peptides from yeast potently induce metabolic insulin actions in isolated rat adipocytes, cardiomyocytes, and diaphragms" ENDOCRINOLOGY, vol. 138, no. 8, 1997, pages 3459-75, XP001017984
- BAESCHLIN, D.K. ET AL.: "Rapid assembly of oligosaccharides: total synthesis of a glycosylphosphatidylinositol anchor of Trypanosoma brucei" ANGEW. CHEM. INT. ED., vol. 37, no. 24, 1999, pages 3423-8, XP001018927
- DEEG, M.A. ET AL: "Inositol glycan phosphate derived from human erythrocyte acetylcholinesterase glycolipid anchor and inositol cyclic 1,2-phosphate antagonize glucagon activation of glycogen phophorylase" DIABATES, vol. 42, no. 9, 1993, pages 1318-23, XP001017977
- SCHMIDT, R.R. ET AL.: "Glycosyl phosphatidylinositol (GPI) anchor synthesis based on versatile building blocks - total synthesis of a GPI anchor of yeast" EUR. J. ORG. CHEM., 1999, pages 1153-65, XP001018004

## Description

### Field of the Invention

The present invention relates to compounds and their uses, and in particular to compounds which have a mimetic or antagonistic property of an inositol phosphoglycan or a free GPI precursor of an IPG, and the uses of these compounds, e.g. to treat a condition ameliorated by administration of an IPG second messenger or an IPG antagonist thereof.

### Background of the Invention

Many of the actions of growth factors and hormones on cells are thought to be mediated by a family of inositol phosphoglycan (IPG) second messengers ^{[13]}. It is thought that the source of IPGs is a "free" form of glycosyl phosphatidylinositol (GPI) situated in cell membranes. IPGs are thought to be released by the action of phosphatidylinositol-specific phospholipases following binding of growth factors to receptors on the cell surface. There is evidence that IPGs mediate the action of a large number of growth factors including insulin, nerve growth factor, hepatocyte growth factor, insulin-like growth factor I (IGF-I), fibroblast growth factor, transforming growth factor β, the action of IL-2 on B-cells and T-cells, ACTH signalling of adrenocortical cells, IgE, FSH and hCG stimulation of granulosa cells, thyrotropin stimulation of thyroid cells, cell proliferation in the early developing ear and rat mammary gland.

Partially characterised inositolphosphoglycans (IPGs) have been postulated to mediate the action of a number of growth factors and hormones including insulin and insulin-like growth factor I (IGF-I) ^{[1]}. Despite their isolation from several tissues type, the precise chemical structures of these IPGs are, however, still unknown and two main structural groups have been proposed on the basis of the chemical composition ^{[2,3]} which display different biological activity and tissue distribution ^{[4]}; the family of glucosamine-*myo*-inositol containing IPGs (IPG-A) and the family of *chiro*-inositol-galactosamine containing IPGs (IPG-P).

In an attempt to establish the minimal structural requirements for biological activity, a number of compounds containing some of the basic structural motifs that have been postulated for IPG mediators have been synthesised in the art ^{[5]}. These synthetic compounds include *O*-(2-amino-2-deoxy-D-glucopyranosyl)-α(1→6)-*chiro*-inositol 1-phosphate and *O*-(2-amino-2-deoxy-D-glucopyranosyl)-α(1→6)-*myo*-inositol 1-phosphate ^{[6]}.

US Patent No: 6,004,938 (Hoechst) discloses a group of synthetic inositol glycans having insulin-like action. The compounds are based on 2-6 monsaccharide units linked to an inositol moiety. The examples in the patent all employ *myo*-inositol and are composed of 5 or 6 units apart from two pseudo-trisaccharide compounds G and H. Compounds G and H are HO-PO(H)O-6Man-α(1→4)-GluN-α(1→6)-(L)inositol-1,2(cyclic) phosphate and HO-PO(H)O-6Man-α(1→4)-GluN-α(1→6)-(L)inositol, otherwise known as *O*-(6-hydrogenphosphonate-α-D-mannopyranosyl)-(1→4)-(2-ammonio-2-deoxy-α-D-glucopyranosyl)-(1→6)-L-*myo*-inositol-1,2-cyclic phosphate and *O*-(6-hydrogenphosphonate-α-D-mannopyranosyl)-(1→4)-(2-amino-2-deoxy-α-D-glucopyranosyl)-L-*myo*-inositol. The properties of exemplified compounds are investigated in lipogenesis and glucose transport assays employing rat fat cells.

WO96/14075 (University of Virginia) discloses a generic family of compounds D-hexosamines linked to an inositol via a β1,4-linkage. The inositols can be *myo* or *chiro*-inositol or pinitol, while the hexosamines are glucosamine or galactosamine. However, this application describes the synthesis of just two compounds 4-*O*-(2-deoxy-2-amino-β-D-galactopyranosyl)-D-pinitol and 4-*O*-(2-deoxy-2-amino-β-D-galactopyranosyl)-D-*chiro*-inositol, or in IUPAC notation *O*-(2-amino-2-deoxy-β-D-galactopyranosyl)-(1→4)-D-pinitol and *O*-(2-amino-2-deoxy-β-D-galactopyranosyl)-(1→4)-D-chiro-inositol.

WO99/06421 (University of Virginia) describes synthetic insulin mimetic substances and includes a general formula I showing β1,4-linked disaccharides. However, despite this the compounds synthesised in this application are exactly the same as those disclosed in the applicant's earlier application, WO96/14075.

A multi-step synthesis of a IPG-P mimetic from glucose has been previously reported in Jaramillo et al ^{[6]}, which discloses a compound called C4, 1-D-6-*O*-(2-amino-2-deoxy-α-D-glucopyranosyl)-*chiro*-inositol 1-phosphate. A further synthesis of C4 is described in our co-pending International Patent Application PCT/GB99/03715 (Rademacher Group Limited). Zapata et al ^{[16]} discloses three other compounds C1-C3 which are:
- C1: 1-D-4-*O*-(2-amino-2-deoxy-α-D-glucopyranosyl)-*myo-*inositol 1-phosphate.
- C2: 1-D-6-*O*-(2-amino-2-deoxy-α-D-glucopyranosyl)-*myo*-inositol 1-phosphate.
- C3: 1-D-6-*O*-(2-amino-2-deoxy-α-D-glucopyranosyl)-*myo*-inositol 1,2 cyclic-phosphate.

It remains a significant problem in the art to produce synthetic compounds which can mimic one or more of the activities of inositol phosphoglycans or which act as antagonists of IPGS.

### Summary of the Invention

Broadly, the present invention relates to IPG mimetic and antagonist compounds and to methods of producing the compounds and to their medical uses. The compounds disclosed herein are useful as synthetic mimetics of IPG-P or IPG-A second messengers and/or growth factors or hormones whose action is mediated by IPGs, as synthetic mimetics of free GPI precursors of IPGs, or as competitive antagonists of IPGs. In particular, the present invention is based on surprising finding that compounds comprising a sugar residue linked to a myo-inositol, wherein the sugar residue is substituted with one or more negatively charged groups, such as phosphate or other phosphoryl group or a sulphate group, have IPG or IPG antagonist biological activities.

Accordingly, in a first aspect, the present invention provides a compound represented by the general formula:
X-α1,6-D-myo-inositol
wherein:
X represents a sugar residue;
the sugar residue is substituted with one or more negatively charged groups selected from phosphate -O-P(O)(OH)₂; thiophosphate -O-P(S)(OH)₂;
phosphonate -O-P(O)OHR; thiophosphonate -O-P(S)OHR; -O-P(S)(OH)(SH);
cyclic phosphate; sulphate O-SO₃H, -O-S(O)(OH); and carboxylic acid;
the sugar residue is optionally further substituted with between one and three groups, and the myo-inositol is unsubstituted or is substituted with between one and five groups, the group or groups independently selected from:
   (a) a phosphoryl group which is as phosphate -O-P(O)(OH)₂; thiophosphate -OP(S)(OH)₂; phosphate esters -O-P(O)(OR)₂; thiophosphate esters -OP(S)(OR)₂; phosphonate -O-P(O)OHR; thiophosphonate -O-P(S)OHR; substituted phosphonate -O-P(O)OR₁R₂; substituted thiophosphonate -O-P(S)OR₁R₂; -O-P(S)(OH)(SH); or cyclic phosphate;
   (b) a phosphorus containing compound which is phosphoramidite -O-P(OR)-NR₁R₂ or phosphoramidate -O-P(O)(OR)-NR₁R₂;
   (c) a sulphur group which is -O-S(O)(OH), -SH, -SR, -S(□O)-R, -S(O)₂R, RO-S(O)₂⁻, -O-SO₂NH₂, -O-SO₂R₁R₂ or sulphamide -NHSO₂NH₂;
   (d) an amino group which is -NHR, -NR₁R₂, -NHAc, -NHCOR, -NH-O-COR,-NHSO₃, -NHSO₂R or -N(SO₂R)₂, and/or an amidino group which is -NH-C(=NH)NH₂ and/or an ureido group which is -NH-CO-NR₁R₂ or a thiouriedo group which is -NH-C(S)-NH₂;
   (e) a hydroxy group or substituted hydroxy group which is -OR₃, where R₃ is C₁₋₁₀ unsubstituted or substituted alkyl, e.g. CHF₂ or CF₃, alkoxyalkyl, aryloxyalkyl, cycloalkyl, alkenyl (unsubstituted alkyl), alkylene (C₃₋₇ cycloalkyl), -OCOR, aryl, heteroaryl, acetal, or where two hydroxyl groups are joined as a ketal;
   (f) a halogen substituentwhich is fluorine or chlorine;
   (g) hydrogen to provide a deoxy sugar;
wherein R, R₁ and R₂ are independently hydrogen or C₁₋₁₀ unsubstituted or substituted alkyl or aryl.

The compounds may be provided as racemic or diasteromeric mixtures, resolved or partially resolved optical isomers, and as pharmaceutically acceptable salts, esters and derivatives as discussed in more detail below.

Examples of groups which may be negatively charged depending on the pH conditions include phosphoryl groups such as phosphate -O-P(O)(OH)₂; thiophosphate -O- , P(S)(OH)₂; phosphonate -O-P(O)OHR; thiophosphonate -O-P(S)OHR; -OP(S)(OH)(SH); cyclic phosphate; or sulphur groups such as sulphate O-SO₃H, -O-S(O)(OH); or carboxylate groups such as carboxylic acid.

Preferably, the sugar residue is substituted at one or more positions other than the position of linkage to the myo-inositol. In embodiments disclosed herein, the sugar residue is substituted at positions 3 and/or 4 and/or 6.

Preferably, the sugar residue is a hexose or a pentose, and may be an aldose or a ketose. The sugar residue can be a member of the D or L series and can include amino sugars, deoxy sugars and their uronic acid derivatives. Preferably, where the sugar residue is a hexose, it is selected from the group consisting of glucose, galactose or mannose, or substituted hexose sugar residues such as an amino sugar residue such as hexosamine, galactosamine or glucosamine, and more preferably D-glucosamine (2-amino-2-deoxy-D-glucose) or D-galactosamine (2-amino-2-deoxy-D-galactose).
Preferred pentose sugar residues include arabinose, fucose and ribose. The sugar residue is optionally substituted at one, two, three or four positions, other than the position of linkage to the myo-inositol moiety.

The myo-inositol moiety may be substituted at one or more of the positions other than the position of linkage to the sugar radical. The sugar radical is optionally substituted at one, two, three or four positions other than at the position of linkage to the myo-inositol moiety (the anomeric position). Examples of substituted groups include CF₃, X(CH₂)ₙ-O-(where X is hydrogen, or substituted or unsubstituted alkyl), CHF₂O-. In further embodiments, the myo-inositol may have one or more of the hydroxyl groups through which the substituents described above are linked to the myo-inositol removed so that any substituent(s) are linked to the ring carbon atom. The sugar residue is optionally substituted at one, two or three positions other than at the position of linkage to the inositol moiety.

The linkage position of the sugar residue to the myo-inositol is a 1,6 linkage. The linkage between the units is preferably via one of the oxygen atoms of the myo-inositol moiety. However, this oxygen atom can be replaced one or more times by -CH₂- or -S- groups.

To avoid confusion, the numbering system used herein is clarified with reference to the following structures. Monosaccharide residues and oligosaccharides are named and numbered according to the recommendation proposed by the Joint Commission on Biochemical Nomenclature, International Union of Pure and Applied Chemistry and International Union of Biochemistry and Molecular Biology.

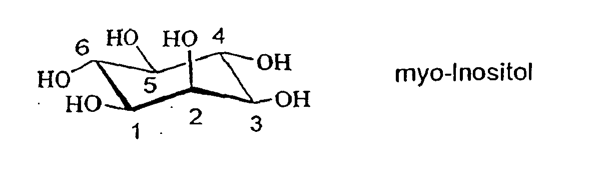

In preferred embodiments, the present invention provides a compound, or a substituted forms and derivatives thereof as defined above, selected from the group consisting of:
- RGL1023: *O*-(2'-amino-2'-deoxy-6'-phosphate-D-glucopyranosyl)-α(1,6)-D-*myo-*inositol.
- RGL1027: *O*-(2'-amino-2'-deoxy-4'-phosphate-D-glucopyranosyl)-α(1,6)-D-*myo-*inositol.
- RGL1029: *O*-(2'-amino-2'-deoxy-3'-phosphate-D-glucopyranosyl)-α(1,6)-D-*myo-*inositol.

- RGL1134: 1'-D-6-*O*-(2'-amino-3'-*O*-benzyl-4'-*O*-phosphate-2'-deoxy-α-D-glucopyranosyl)-3,4,5-tri-*O*-benzyl-*myo*-inositol-1,2-cyclic phosphate.
- RGL1133: 1'-D-6-*O*-(2'-amino-3'-*O*-benzyl-4',6'-di-*O*-sulphate-2'-deoxy-α-D-glucopyranosyl)-3,4,5-tri-O-benzyl-myo-inositol-1,2-di-O-sulphate.
- RGL1135: 1'-D-6-*O*-(2'-amino-3'-*O*-benzyl-4',6'-di-*O*-cyclic phosphate-2'-deoxy-α-D-glucopyranosyl)-3,4,5-tri-*O*-benzyl-*myo*-inositol-1,2-cyclic phosphate.
- RGL 1130: 1'-D-6-*O*-(2'-amino-4'-*O*-phosphate-2'-deoxy-α-D-glucopyranosyl)-*myo*-inositol-1,2-cyclic phosphate.
1'-D-6-*O*-(2'-amino-6'-*O*-phosphate-2'-deoxy-α-D-glucopyranosyl)-*myo*-inositol-1,2-cyclic phosphate.

In a further aspect, the present invention provides methods for making the compounds of the invention or their intermediates as set out in the following experimental description and the schemes. In a further related aspect, the present invention further relates to compounds which are the novel intermediates described herein.

In a further aspect, the present invention provides one or more of the above compounds for use in a method of medical treatment. The compounds may be useful as IPG mimetics or IPG antagonists, e.g. as competitive antagonists.

In a further aspect, the present invention provides the use of one or more of the above compounds for the preparation of a medicament for the treatment of a condition ameliorated by the administration of an inositol phosphoglycan (IPG) second messenger or an IPG antagonist. Examples of such conditions are set out in the pharmaceutical uses section below.

Embodiments of the invention will now be described by way of example and not limitation with reference to the accompanying drawings.

### Brief Description of the Figures

Scheme 1 shows the coupling of diol 2 with trichloroacetimidate **1** resulting in 6-*O-*glycosylation. Subsequent manipulation of protective groups afforded compound **4.**

Scheme 2 shows the production of compounds RGL1023 and RGL1027 from intermediate **4**.

Scheme 3 shows the production of compound RGL1029 by coupling acceptor **2** with trichloroacetimidate **11**.

Scheme 5 shows the synthesis of RGL1134.

Scheme 6 shows the synthesis of RGL1135.

Scheme 7 shows the synthesis of RGL1133.

Scheme 8 show the synthesis of RGL1130.

Figure 1 shows a graph of the PDH phosphatase activation of exemplary compounds of the invention as compared to *myo*-inositol.

Figure 2 shows a graph of glucose stimulated lipogenesis of exemplary compounds of the invention.

Figure 3 shows a graph of insulin stimulated lipogenesis of exemplary compounds of the invention.

Figure 4 shows a graph of the PKA inhibition of exemplary compounds of the invention.

Figure 5 shows the results of a G6Pase inhibition assay testing the effect of compound RGL 1133 against a known inhibitor, sodium O-vanadate.

### Detailed Description

### Inositol phosphoglycans (IPGs)

IPG-A mediators modulate the activity of a number of insulin-dependent enzymes such as cAMP dependent protein kinase (inhibits), adenylate cyclase (inhibits) and cAMP phospho-diesterases (stimulates). In contrast, IPG-P mediators modulate the activity of insulin-dependent enzymes such as pyruvate dehydrogenase phosphatase (stimulates) and glycogen synthase phosphatase (stimulates). The A-type mediators mimic the lipogenic activity of insulin on adipocytes, whereas the P-type mediators mimic the glycogenic activity of insulin on muscle. Both A-and P-type mediators are mitogenic when added to fibroblasts in serum free media. The ability of the mediators to stimulate fibroblast proliferation is enhanced if the cells are transfected with the EGF-receptor. A-type mediators can stimulate cell proliferation in the chick cochleovestibular ganglia.

Soluble IPG fractions having A-type and P-type activity have been obtained from a variety of animal tissues including rat tissues (liver, kidney, muscle, brain, adipose, heart) and bovine liver. IPG-A and IPG-P biological activity has also been detected in human liver and placenta, malaria parasitized RBC and mycobacteria. The ability of an anti-inositolglycan antibody to inhibit insulin action on human placental cytotrophoblasts and BC3H1 myocytes or bovine-derived IPG action on rat diaphragm and chick cochleovestibular ganglia suggests cross-species conservation of many structural features. However, it is important to note that although the prior art includes these reports of IPG-A and IPG-P activity in some biological fractions, the purification or characterisation of the agents responsible for the activity is not disclosed.

IPG-A substances are cyclitol-containing carbohydrates, also containing Zn²⁺ ions and phosphate and having the properties of regulating lipogenic activity and inhibiting cAMP dependent protein kinase. They may also inhibit adenylate cyclase, be mitogenic when added to EGF-transfected fibroblasts in serum free medium, and stimulate lipogenesis in adipocytes.

IPG-P substances are cyclitol-containing carbohydrates, also containing Mn²⁺ and/or Zn²⁺ ions and phosphate and having the properties of regulating glycogen metabolism and activating pyruvate dehydrogenase phosphatase. They may also stimulate the activity of glycogen synthase phosphatase, be mitogenic when added to fibroblasts in serum free medium, and stimulate pyruvate dehydrogenase phosphatase.

Methods for obtaining A-type and P-type mediators are set out in Caro et al, 1997, and in WO98/11116 and WO98/11117. Protocols for determining characteristic IPG biological activities such as PDH activation, PKA inhibition, acetylCoA activation, fructose-1,6-bis-phosphatase activity and lipogenesis are well known in the art, e.g. as described in Caro et al ^{[14]}.

### Drug Formulation

The compounds of the invention may be derivatised in various ways. As used herein "derivatives" of the compounds includes salts, coordination complexes with metal ions such as Mn²⁺ and Zn²⁺, esters such as *in vivo* hydrolysable esters, free acids or bases hydrates or glycolipid derivatives.

Salts of the compounds of the invention are preferably physiologically well tolerated and non toxic. Many examples of salts are known to those skilled in the art.
Compounds having acidic groups, such as phosphates or sulfates, can form salts with alkaline or alkaline earth metals such as Na, K, Mg and Ca, and with organic amines such as triethylamine and Tris (2-hydroxyethyl)amine. Salts can be formed between compounds with basic groups, e.g. amines, with inorganic acids such as hydrochloric acid, phosphoric acid or sulfuric acid, or organic acids such as acetic acid, citric acid, benzoic acid, fumaric acid, or tartaric acid. Compounds having both acidic and basic groups can form internal salts.

Esters can be formed between hydroxyl or carboxylic acid groups present in the compound and an appropriate carboxylic acid or alcohol reaction partner, using techniques well known in the art.

Some of the above defined derivatives may act as prodrugs of the compounds are convertible *in vivo* or *in vitro* into one of the parent compounds. Typically, at least one of the biological activities of compound will be reduced in the prodrug form of the compound, and can be activated by conversion of the prodrug to release the compound or a metabolite of it.

An example of these derivatives are glycolipid derivatives in which one or more lipid moieties are provided as substituents on the sugar residue or the cyclitol moieties, leading to the release of the free form of the compound by cleavage with a phospholipase enzyme.

### Pharmaceutical Compositions

The compounds described herein or their derivatives can be formulated in pharmaceutical compositions, and administered to patients in a variety of forms, in particular to treat conditions which are ameliorated by the administration of inositol phosphoglycan second messengers or IPG antagonists such as competitive antagonist.

Pharmaceutical compositions for oral administration may be in tablet, capsule, powder or liquid form. A tablet may include a solid carrier such as gelatin or an adjuvant or an inert diluent. Liquid pharmaceutical compositions generally include a liquid carrier such as water, petroleum, animal or vegetable oils, mineral oil or synthetic oil. Physiological saline solution, or glycols such as ethylene glycol, propylene glycol or polyethylene glycol may be included. Such compositions and preparations generally contain at least 0.1 wt% of the compound.

Parental administration includes administration by the following routes: intravenous, cutaneous or subcutaneous, nasal, intramuscular, intraocular, transepithelial, intraperitoneal and topical (including dermal, ocular, rectal, nasal, inhalation and aerosol), and rectal systemic routes. For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, solutions of the compounds or a derivative thereof, e.g. in physiological saline, a dispersion prepared with glycerol, liquid polyethylene glycol or oils.

In addition to one or more of the compounds, optionally in combination with other active ingredient, the compositions can comprise one or more of a pharmaceutically acceptable excipient, carrier, buffer, stabiliser, isotonicizing agent, preservative or anti-oxidant or other materials well known to those skilled in the art. Such materials should be non-toxic and should not interfere with the efficacy of the active ingredient. The precise nature of the carrier or other material may depend on the route of administration, e.g. orally or parentally.

Liquid pharmaceutical compositions are typically formulated to have a pH between about 3.0 and 9.0, more preferably between about 4.5 and 8.5 and still more preferably between about 5.0 and 8.0. The pH of a composition can be maintained by the use of a buffer such as acetate, citrate, phosphate, succinate, Tris or histidine, typically employed in the range from about 1 mM to 50 mM. The pH of compositions can otherwise be adjusted by using physiologically acceptable acids or bases.

Preservatives are generally included in pharmaceutical compositions to retard microbial growth, extending the shelf life of the compositions and allowing multiple use packaging. Examples of preservatives include phenol, meta-cresol, benzyl alcohol, para-hydroxybenzoic acid and its esters, methyl paraben, propyl paraben, benzalconium chloride and benzethonium chloride. Preservatives are typically employed in the range of about 0.1 to 1.0 % (w/v).

Preferably, the pharmaceutically compositions are given to an individual in a "prophylactically effective amount" or a "therapeutically effective amount" (as the case may be, although prophylaxis may be considered therapy), this being sufficient to show benefit to the individual. Typically, this will be to cause a therapeutically useful activity providing benefit to the individual. The actual amount of the compounds administered, and rate and time-course of administration, will depend on the nature and severity of the condition being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. (ed), 1980. By way of example, and the compositions are preferably administered to patients in dosages of between about 0.01 and 100mg of active compound per kg of body weight, and more preferably between about 0.5 and 10mg/kg of body weight.

The composition may further comprise one or more other pharmaceutically active agents, either further compounds of the invention, inositol phosphoglycans, growth factors such as insulin, NGF or other growth factors listed below; or other drugs, e.g. those in use for the treatment of diabetes or other conditions set out below.

### Medical Uses

As set out above, IPGs are second messengers for a range of different growth factors, including insulin, nerve growth factor, hepatocyte growth factor, endothelial growth factor (EGF), insulin-like growth factor I (IGF-I), fibroblast growth factor, transforming growth factor β, the action of IL-2 on B-cells and T-cells, ACTH signalling of adrenocortical cells, IgE, FSH and hCG stimulation of granulosa cells, thyrotropin stimulation of thyroid cells, cell proliferation in the early developing ear and rat mammary gland. Consequently, IPGs or their antagonists can be used in the treatment or amelioration of disorders mediated by the growth factors or to mimic specific growth factor biological activities.

Examples of conditions which can be treated using IPGs or IPG antagonists include, diabetes, obesity, dyslipidaemia, pre-eclampsia, neurotrophic disorders, hepatic damage and adrenal atrophy.

WO98/10791 discloses that pre-eclampsia is characterised by elevated levels of IPG-P and that it can be treated using an IPG-P antagonist. Compounds of the invention which are IPG-P antagonists, e.g. antagonists which compete with wild-type IPG-P but lack one or more of its activities, could be used in the treatment of pre-eclampsia.

The use of both IPG-P and IPG-A and IPG-A antagonists in the diagnosis and treatment of diabetes is disclosed in WO98/11435. This application discloses that in some forms of diabetes the ratio of P:A-type IPGs is imbalanced and can be corrected by administering a medicament containing an appropriate ratio of IPG-P, IPG-A or antagonist(s) thereof. In particular, it describes the treatment of obese type II diabetes (NIDDM) patients with a P-type IPG and/or an A-type IPG antagonist and the treatment of IDDM or lean type II diabetes (body mass index < 27) with a mixture of P- and A-type IPGs, typically in a P:A ratio of about 6:1 for males and 4:1 for females. The compounds and compositions of the present invention can be employed in such types of treatment. More particularly, the compounds are likely to be of use in the treatment of various form of diabetes and diabetic complications including diabetes due to insulin resistance, insulin resistance in type I diabetes and brittle diabetes, obese or lean type II diabetes, and of conditions associated with insulin resistance or insulin underproduction, such as neurotrophic disorders or polycystic ovary syndrome, lipodystrophy, age-related memory loss, and post-ischaemic damage secondary to stroke or post-transplant complications.

The compounds of this invention are also likely to be of use in controlling neuron proliferation or neurite outgrowth, either *in vitro* or *in vivo,* e.g. acting as a nerve or neurite growth factor mimetic second messenger. They may thus have applications in the treatment and/or diagnosis of any condition related to neuron proliferation or neurite differentiation. WO99/38516 discloses that IPG-A and synthetic mimetics thereof cause neuron proliferation, mimicking the action of the growth factor IGF-I. In contrast, IPG-P and synthetic mimetics thereof such as compound C4 cause neurite outgrowth. The neurons may be central (brain and spinal cord) neurons, peripheral (sympathetic, parasympathetic, sensory and enteric) neurons, e.g. in the regeneration of peripheral nerves, or motor neurons. Treatments may involve the treatment of damage to nerve, spinal cord or central nervous system damage secondary to trauma, or autoimmune or metabolic damage, or post-ischaemic damage secondary to stroke or post-transplant complications, motor neuron disease, neurodegenerative disorders or neuropathy. Damage to the nervous system includes the results of trauma, stroke, surgery, infection (e.g. by viral agents), ischemia, metabolic disease, toxic agents, or a combination of these or similar causes. Motor neuron disease includes conditions involving spinal muscular atrophy, paralysis or amyotrophic lateral sclerosis. Neurodegenerative disorders include Parkinson's disease, Alzheimer's disease, epilepsy, multiple sclerosis, Huntingdon's chorea and Meniere's disease.

The compounds of the invention may also be useful as hepatocyte growth factor mimetic second messengers, e.g. in the preparation of medicaments for the treatment of hepatic damage caused by infection, alcohol abuse, drug sensitivity, or autoimmunity. The compounds may also be useful as fibroblast growth factor mimetic second messengers or epidermal growth factor mimetic second messengers, e.g. in the preparation of medicaments for the promotion of wound healing following surgery or trauma or tissue damage induced by ischaemia or autoimmunity.

In other embodiments, the compounds of the invention may be useful as adrenal cell growth factor mimetic second messengers or ACTH mimetic second messengers in the preparation of a medicament for the treatment of disease states involving adrenal atrophy.

The compounds of the invention can readily be tested using the assays identified herein to determine their suitability for some or all of the medical uses described above. Thus, even compounds with a relatively low activity in one of the enzymes assays disclosed herein, may be useful by virtue of possessing a different activity, and moreover the pattern of activities can be used to rapidly screen the compounds for suitability in the various medical applications disclosed herein.

| Activity | Diabetes I | Diabetes II | Obesity | Alzheimer's | Neurotrophics |
|---|---|---|---|---|---|
| PDH Kinase | Inhibit | Inhibit | No Effect | Inhibit | No effect |
| PDH Phosphatase | Activate | Activate | No effect | No effect | No effect |
| Acetyl CoA carboxylase I * | Activate | No effect | No effect | No effect | No effect |

| | | | | | |
|---|---|---|---|---|---|
| * found in liver and adipose cytosol. | | | | | |

### Methods of Making the Compounds

Based on the disclosure herein, the knowledge in the art and in references ^{[5-11]}, the skilled person could couple sugar residues and myo-inositols together, optionally with one or more substituents.

Useful guidance on the synthesis of the exemplified compounds and for introducing the substituents set out herein is provided by the papers by Gigg & Gigg, Khiar & Martin-Lomas ^{[5]} and Baeschlin et al ^{[18]} and the references cited therein.

Phosphoryl groups such as phosphate, cyclic phosphate or substituted phosphate or cyclic phosphate can be substituted into the compounds of the invention by the phosphate or phosphoramidite method, Bannwath et al, Helvetica Chemica Acta, 70:175-186, 1987 and Yu & Fraser-Reid, Tetrahedron Lett., 29:979-982, 1988.

Phosphate protecting groups can also be synthesized according to the methods disclosed in Hoeben-Weyl, Methods of Organic Chemistry, volume 12/1 or 12/2, Teilheimer, Synthetic Methods of Organic Chemistry, Vol 45. Protecting groups for the OH of sugars include menthoxycarbonyl (MntCO), acetal (in particular, two R groups may together represent a bridging acetal such as *O*-cyclohexylidene, *O-*isopropylidene or *O*-benzylidene), *tert*-butyldimethylsilyl (TBDMS), benzyl (Bn), *tert*-butyldiphenylsilyl (TBDPS). Many protecting groups suitable for use in the syntheses and reactions of saccharides are known and are well documented in standard reference works. The choice depends in part on the route by which the compound is synthesised and/or on the uses to which it is to be put, including the reactions which it is subsequently intended to undergo.

### Bioactivity Assays

The compounds of the invention can be tested for one or more the characteristic IPG-P and/or IPG-A activities mentioned above to determine whether they will be suitable for use a IPG mimetics or antagonists. Preferred assays measure the effect of the compounds on PDH phosphatase, PKA or lipogenesis. Protocols for these assays are provided in Caro et al ^{[14]}. The compounds can also be tested to determine whether they activate or inhibit other enzymes involved in insulin signalling mechanism, such as glucose-6-phosphatase.

### Examples

### General Methods

All reactions were carried out under an atmosphere of dry argon using oven-dried glassware and freshly distilled and dried solvents. THF and diethyl ether were distilled from sodium benzophenone ketyl. Dichloromethane and acetonitrile were distilled from calcium hydride. TLC was performed on Silica gel GF₂₅₄ (Merck) with detection by charring with phosphomolibdic acid/EtOH. For flash chromatography, Silica Gel (Merck 230-400 mesh) was used. Columns were eluted with positive air pressure.
Chromatographic eluents are given as volume to volume ratios (v/v). Routine NMR spectra were recorded with Bruker Avance DPX300 (¹H, 300 MHz), Bruker Avance DRX400 (¹H, 400 MHz), and Bruker Avance DRX500 (¹H, 500 MHz) spectrometers. Chemical shifts are reported in ppm, and coupling constants are reported in Hz. Spectra were referenced to the residual proton or carbon signals of the solvent. High-resolution mass spectra were recorded on a Kratos MS-80RFA 241-MC apparatus.
Optical rotations were determined with a Perkin-Elmer 341 polarimeter. Elemental analyses were performed using a Leco CHNS-932 apparatus. The organic extracts were dried over anhydrous sodium sulfate and concentrated *in vacuo.* The synthesis of compounds RGL1023, 1027 and 1029 involved the preparation of a glycosyl acceptor with position 6 free for reaction with the corresponding glycosyl donor. Diol **2** was chosen as the *myo*-inositol acceptor ^{[17]} as it can be regioselectively glycosylated at position 6. Regio- and stereoselective glycosylation of diol 2 is most conveniently performed using the trichloroacetimidate procedure with 2-azido-2-deoxy glycosyl donors bearing protective group patterns compatible with the further transforamtions required and designed as to provide an acceptable reactivity-selectivity balance in the forthcoming glycosylation reaction. Thus, use of trichloroacetimidate 1 was the glycosyl donor of choice. Coupling of 1 with 2 in diethyl ether at -20°C using TMS triflate as promoter resulted in 6-*O*-glycosylation with good yield. Manipulation of protective groups afforded compound 4, a key intermediate for compounds RGL 1023 and RGL 1027 (Scheme 1). Selective opening of the benzylidene protecting group on positions 4' and 6' of the hexosamine residue afforded **5** and **6** in good yields. These compounds, precursors of phosphorylated species RGL 1023 and RGL 1027, were subjected to treatment with phosphorylating reagent dibenzyloxi(diisopropylamino)phosphine, which afforded the corresponding phosphite derivatives. Oxidation to the phosphate form was achieved *in situ* by reaction with MPBCA in dichloromethane at room temperature for two hours with good yield. Trifluoracetic acid treatment of compounds 7 and 8 in dichloromethane at room temperature for 4 hours afforded diols 9 and 10 with moderate to good yields. Compounds RGL1023 and RGL1027 were obtained after hydrogenolytic debenzylation and concomitant azide reduction of **9** and **10** in buffered medium (Scheme 2).

Synthesis of compound RGL1029 required that the glycosyl donor to be used contained a protecting group in position 3 different from a benzyl group, to be introduced later on in the glycosyl acceptor residue. Thus, trichloroacetimidate **11,** bearing a p-methoxy-benzyl group in position 3 was used. Coupling with acceptor 2 in the conditions described above afforded compound **12** in good yield. Trifluoracetic acid hydrolysis and subsequent treatment with benzyl chloride in the presence of NaH in DMF afforded the benzylated derivative **13,** which afforded precursor 14 in good yield after oxidative cleavage of the p-methoxy-benzyl group with 2,3-dichloro-5,6-dicyano-1,4-benzoquinone in dichloromethane.

Treatment of compound 14 with dibenzyl(diisopropyl)phosphoramidite followed by oxidation of the phosphite group afforded compound 15 (75%). Deprotection of the ketal group on the inositol moiety was achieved by acid hydrolysis to yield compound 16 (65%), which was then subjected to catalytic hydrogenolysis to produce RGL1029 quantitatively.

### Synthesis of RGL 1023, 1027 and 1029

### 1'-D-6-O-(2'-azido-3'-O-benzyl-4',6'-O-benzylidene-2'-deoxy-α-D-glucopyranosyl)-1,2-O-(L-1,7,7-trimethyl[2.2.1]-bicyclohept-2-ylidene)-3,4-O-(1,1,3,3-tetraisopropyldisiloxanyl)-myo-inositol (3)

To a solution of trichloroacetimidate 2-azido-3-*O*-benzyl-4,6-*O*-benzylidene-2-deoxy-D-glucopyranoside **1** (1.698 g, 3.217 mmol) and 1-D-1,2-*O*-(L-1,7,7-trimethyl[2.2.1]-bicyclohept-2-ylidene)-3,4-*O*-(1,1,3,3-tetraisopropyldisiloxanyl)-*myo*-inositol **2** (2.51 g, 4.507 mmol) in Et₂O (50 mL) at -20°C, trimethylsilyl trifluromethanesulfonate (29 ml, 0.160 mmol) was added. The mixture was warmed to room temperature over 5 h, quenched by addition of Et₃N (0.5 mL), filtered through celite and concentrated. The residue was purified by flash chromatography (Hexane/EtOAc 95/5 to 75/25) to give three disaccharides : α(1→6) **3** as a white solid (55% yield).

### 1'-D-6-O-(2'-azido-3'-O-benzyl-4',6'-O-benzylidene-2'-deoxy-α-D-glucopyranosyl)-1,2-O-(L-1,7,7-trimethyl[2.2.1]-bicyclohept-2-ylidene)-3,4,5-triO-benzyl-myo-inositol (4)

To a solution of disaccharide α(1-6) **3** (1.60 g, 1.735 mmol) in THF (20 mL) at 0°C, tetrabutyl ammonium fluoride was added (1 M solution in THF, 3.81 mL). After 15 min the solution was concentrated and the residue re-dissolved in DMF (15 mL). The solution was cooled to 0°C and 60 % sodium hydride in mineral oil (312 mg, 7.801 mmol) and benzyl bromide (0.93 mL, 7.801 mmol) was added. After 2 h methanol was added. The mixture was diluted with CH₂Cl₂ (100mL), washed with sat. NaCl (2x100 mL), dried over MgSO₄ and concentrated. Flash chromatography of the crude (hexane/AcOEt 95/5) gave product **4** (1.410 g, 1.484 mmol, 85% over two steps).

### 1'-D-6-O-(2'-azido-3',6'-di-O-benzyl-2'-deoxy-α-D-glucopyranosyl)-1,2-O-(L-1,7,7-trimethyl[2.2.1]-bicyclohept-2-ylidene)-3,4,5-tri-O-benzyl-myo-inositol (5)

To a solution of disaccharide 4 (1.363 g, 1.431 mmol) in THF (40 mL) sodium cyanoborohydride (1M solution in THF, 21.5 mL) was added. After 15 min at r.t. hydrochloric acid (1M solution in ether) was added until evolution of hydrogen ceased. The mixture was stirred at r.t. for 2 h, diluted with CH₂Cl₂ (60 mL) and washed with sat. NaHCO₃ (2x100 mL). The aqueous layer was extracted with CH₂Cl₂ (2x50 mL) and the combined organic layer was dried over MgSO₄ and concentrated. Flash chromatography of the crude (hexane/AcOEt 9/1) gave the compound 5 (1.0 g, 1.050 mmol, 73 %).

### 1'-D-6-O-(2'-azido-3',4'-di-O-benzyl-2'-deoxy-α-D-glucopyranosyl)-1 ,2-O-(L-1,7,7-trimethyl[2.2.1]-bicyclohept-2-ylidene)-3,4,5-tri-O-benzyl-myo-inositol (6)

To a solution of disaccharide 4 (460 mg, 0.484 mmol) and borane-dimethylamine complex (115 mg, 1.952 mmol) in CH₂Cl₂(40 mL) at 0°C, boron trifluoride diethyl etherate (254 mL, 1.963 mmol) was added dropwise. After 30 min, the stirring was continued at r.t. for 1 hour, and then the reaction quenched with sat. NaHCO₃ (15 mL). The crude material was diluted with CH₂Cl₂ (60 mL), washed with sat. NaCl (3x100 mL), dried over MgSO₄ and concentrated. Flash chromatography of the crude (hexane/AcOEt 6/1) gave compound 6 (276 mg, 0.290 mmol, 60 %).

### 1'-D-6-O-(2'-azido-3',6'-di-O-benzyl-2'-deoxy-4'-dibenzyl-phosphate-α-D-glucopyranosyl)-1,2-O-(L-1,7,7-trimethyl[2.2.1]-bicyclohept-2-ylidene)-3,4,5-triO-benzyl-myo-inositol (7)

To a solution of disaccharide **5** (100 mg, 0.105 mmol) and 1-H-tetrazole (30 mg, 0.428 mmol) in anh. CH₂Cl₂(10 mL) at 0°C, dibenzyl diisipropylphosphoramidite (141 mL, 0.420 mmol) was added dropwise. After the addition was completed, the icebath was removed and the solution stirred for 2 h 30 min. The mixture was cooled to -40°C and a solution of 70% 3-chloroperbenzoic acid (65 mg, 0.264 mmol) in CH₂Cl₂ (4 mL) was added. The mixture was stirred for 2 h 30 min, diluted with CH₂Cl₂ (30 mL), washed with sat. Na₂SO₃ (2x50 mL), sat. NaHCO₃ (2x50 mL) and sat. NaCl (2x50 mL), dried over MgSO₄ and concentrated. Flash chromatography of the crude mixture (hexane/AcOEt 4/1) gave compound 7 (100 mg, 0.082 mmol, 78%).

### 1'-D-6-O-(2'-azido-3',4'-di-O-benzyl-2'-deoxy-6'-dibenzyl-phosphate-α-D-glucopyranosyl)-1,2-O-(L-1,7,7-trimethyl[2.2.1]-bicyclohept-2-ylidene)-3,4,5-triO-benzyl-myo-inositol (8)

To a solution of disaccharide **6** (150 mg, 0.157 mmol) and 1-H-tetrazole (44 mg, 0.628 mmol) in CH₂Cl₂(15 mL) at 0°C, dibenzyl diisipropylphosphoramidite (212 mL, 0.630 mmol) was added dropwise. After the addition was completed, the solution was stirred at r.t. for 3 h. The mixture was cooled to -40°C and a solution of 70% 3-chloroperbenzoic acid (97 mg, 0.393 mmol) in anh. CH₂Cl₂ (5 mL) was added. The mixture was stirred for 45 min, diluted with CH₂Cl₂ (30 mL), washed with sat. NaHSO₃ (2x50 mL), sat. NaHCO₃ (2x50 mL) and sat. NaCl (2x50 mL), dried over MgSO₄ and concentrated. Flash chromatography of the crude mixture (hexane/AcOEt 4/1) gave compound **8** (152 mg, 0.125 mmol, 80%).

### 1'-D-6-O-(2'-azido-3',6'-di-O-benzyl-2'-deoxy-4'-dibenzyl-phosphate-α-D-glucopyranosyl)-3,4,5-tri-O-benzyl-myo-inositol (9)

To a solution of disaccharide **7** (100 mg, 0.082 mmol) in CH₂Cl₂ (10 mL) H₂O (0.09 mL, 5 mmol), and trifluoroacetic acid (0.38 mL, 4.949 mmol) were added and the reaction stirred for 4 h at r.t. The mixture was then diluted with CH₂Cl₂ (40 mL), washed with sat. NaHCO₃ (2x50 mL), sat. NaCl (3x50 mL), dried over MgSO₄ and concentrated. Flash chromatography of the crude mixture(hexane/AcOEt 1/1 to 1/2 and finally AcOEt 100%) gave compound **9** (60 mg, 0.056 mmol, 68%).

### 1'-D-6-O-(2'-azido-3',4'-di-O-benzyl-2'-deoxy-6'-dibenzyl-phosphate-α-D-glucopyranosyl)-3,4,5-tri-O-benzyl-myo-inositol (10)

To a solution of disaccharide **8** (110 mg, 0.091 mmol) in CH₂Cl₂ (10 mL) H₂O (0.1 mL, 5.551 mmol), and trifluoroacetic acid (0.42 mL, 5.470 mmol) were added and the reaction stirred for 4 h at r.t. The mixture was then diluted with CH₂Cl₂ (40 mL), washed with sat. NaHCO₃ (2x50 mL), sat. NaCl (3x50 mL), dried over MgSO₄ and concentrated. Flash chromatography of the crude mixture (hexane/AcOEt 1/1 to 1/2 and finally AcOEt 100%) gave compound **10** (84 mg, 0.078 mmol, 86%) as a white solid.

### 1'-D-6-O-(2'-amino-2'-deoxy-4'-phosphate-α-D-glucopyranosyl)-myo-inositol (RGL1027)

To a suspension of disaccharide **9** (16 mg, 14.840 mmol) in EtOH (0.6 mL) 10% Pd/C (3.2 mg, 0.003 mmol) was added and the reaction stirred under hydrogen atmosphere at r.t. for 36 h. The solvent was evaporated, the crude suspended in H₂O dest., filtered through celite and the filtrate lyophilized to give **RGL1027** (4.6 mg, 10.919 mmol, 74%). ¹H-NMR (D₂O, 500 MHz): δ 5.25 (broad s, 1H, H₁,), 3.96 (m, 1H, H_{4'}), 3.91 (m, 2H, H_{3'}+H₂), 3.84 (m, 1H, H_{5'}), 3.82 (m, 1H, H_{6'b})_{,} 3,61 (m, 3H, H_{6'a}+H₁+H₆), 3.52 (t, *J*= 8.85 Hz, 1H, H₄), 3.41 (broad d, *J*= 8.85 Hz, 1H, H₃), 3.27 (d, *J*= 8.85 Hz, 1H, H₅), 3.18 (m, 1H, H_{2'}). ¹³C-NMR (D₂O, 500 MHz): d 97.40 (C_{1'}), 80.94 (C₆), 73.03 (C₅), 72.91 (C₄), 72.68 (C₂), 72.25 (C_{5'}), 72.08 (C₁), 71.99 (C_{4'}), 71.32 (C₃), 70.79 (C_{3'}), 60.36 (C_{6'a}+C_{6'b}), 55.07 (C_{2'}).

### 1'-D-6-O-(2'-amino-2'-deoxy-6'-phosphate-α-D-glucopyranosyl)-myo-inositol (RGL1023)

To a suspension of disaccharide **10** (16 mg, 14.840 mmol) in a mixture MeOH/H₂O/AcOH 9/1/0.1 (0.5 mL) 10% Pd/C (3.2 mg, 0.003 mmol) was added, and the reaction stirred under hydrogen atmosphere at r.t. for 18 h. The crude was filtered through celite with an aqueous wash and the filtrate lyophilized to give **RGL1023** (5.5 mg, 13.055 mmol, 88%). ¹H-NMR (D₂O, 500 MHz): δ 5.23 (broad s, 1H, H_{1'}), 4.00 (m, 1H, H_{5'}), 3.99 (m, 1H, H_{6'a}), 3.90 (broad s, 1H, H₂), 3.78 (m, 1H, H_{6'b})_{,} 3.73 (broad t, *J* = 9.1 Hz, 1H, H_{3'}), 3.61 (m, 2H, H₁+H₆), 3.60 (m, 1H, H_{4'}), 3.53 (t, *J*= 9.1 Hz, 1H, H₄), 3.40 (dd, *J*₁= 2.3 Hz, *J*₂= 9.1 Hz, 1H, H₃), 3.26 (broad t, *J* = 8.9 Hz, 1H, H₅), 3.14 (m, 1H, H_{2'}). ¹³C-NMR (D₂O, 500 MHz): d 97.49 (C₁), 81.14 (C₆), 73.69 (C₅), 72.90 (C₄), 72.72 (C₂), 72.69 (C_{5'}), 72.0 (C₁), 71.27 (C₃), 69.19 (C_{4'}), 62.5 (C_{6'a}+C_{6'b}), 56.65 (C_{3'}), 55.0 (C_{2'}).

### 1'-D-6-O-(2'-azido-3'-O-(para)methoxybenzyl-4',6'-O-benzylidene-2'-deoxy-α-D-glucopyranosyl)-1,2-O-(L-1,7,7-trimethyl[2.2.1]-bicyclohept-2-ylidene)-3,4-O-(1,1,3,3-tetraisopropyldisiloxanyl)-myo-inositol (12)

To a solution of trichloroacetimidate 2-azido-3-*O*-(*para*)methoxybenzyl-4,6-*O-*benzylidene-2-deoxy-D-glucopyranoside **11** (166 mg, 0.298 mmol) and 1-D-1,2-*O*-(L-1,7,7-trimethyl[2.2.1]-bicyclohept-2-ylidene)-3,4-*O*-(1,1,3,3-tetraisopropyldisiloxanyl)-*myo*-inositol **2** (166 mg, 0.298 mmol) in Et₂O (3 mL) at r.t., trimethylsilyl trifluoromethanesulfonate (1 mL, 5.534 mmol) was added. After 20 minutes, the reaction was quenched by addition of Et₃N (0.1 mL), filtered through celite, concentrated and purified by flash chromatography (hexane/AcOEt 95/5) to give disaccharide **12** (171 mg, 0.180 mmol, 60%).

### 1'-D-6-O-(2'-azido-3'-O-paramethoxybenzyl-4',6'-O-benzylidene-2'-deoxy-α-D-glucopyranosyl)-1,2-O-(L-1,7,7-trimethyl[2.2.1]-bicyclohept-2-ylidene)-3,4,5-triO-benzyl-myo-inositol (13)

To a solution of disaccharide **12** (120 mg, 0.126 mmol) in THF (1.5 mL) at 0°C, tetrabutylammonium fluoride (1M solution in THF, 284 mL) was added. The solution was warmed up to r.t. over 1 hour and then concentrated. The residue was re-dissolved in DMF (2 mL), cooled to 0°C and 60% sodium hydride in mineral oil (23 mg, 0.575 mmol) and benzyl bromide (67 mL, 0.563 mmol) were added. After stirring at r.t. overnight under argon atmosphere, the excess of base was destroyed by addition of methanol, the mixture concentrated to dryness, diluted with CH₂Cl₂ (25 mL), washed with sat. NaCl (3x25 mL), dried over MgSO₄ and the solvent evaporated to dryness. Flash chromatography of the crude mixture (hexane/AcOEt 9:1) compound **13** (98 mg, 0.100 mmol, 79%).

### 1'-D-6-O-(2'-azido-4',6'-O-benzylidene-2'-deoxy-α-D-glucopyranosyl)-1,2-O-(L-1,7,7-trimethyl[2.2.1]-bicyclohept-2-ylidene)-3,4,5-tri-O-benzyl-myo-inositol (14)

To a solution of disaccharide **13** (120 mg, 0.122 mmol) in a CH₂Cl₂/H₂O 9/1 mixture (1.5 mL), 2,3-dichloro-5,6-dicyano-1,4-benzoquinone (35 mg, 0.154 mol) was added, and stirred for 30 min at r.t. The reaction was diluted with CH₂Cl₂ (25 mL), filtered over celite, washed with sat. NaHCO₃ (2x25 mL), sat. NaCl (25 mL), dried over Na₂SO₄ and concentrated. Flash chromatography of the crude mixture (hexane/AcOEt 6/1) gave dissaccharide **14** (87 mg, 0.101 mmol , 83%).

### 1'-D-6-O-(2'-azido-4',6'-O-benzylidene-3'-dibenzyl-phosphate-2'-deoxy-α-D-glucopyranosyl)-1,2-O-(L-1,7,7-trimethyl[2.2.1]-bicyclohept-2-ylidene)-3,4,5-triO-benzyl-myo-inositol (15)

To a solution of disaccharide **14** (70 mg, 0.081 mmol) and 1-H-tetrazole (23 mg, 0.328 mmol) in CH₂Cl₂ (7 mL) at 0°C, dibenzyl diisipropylphosphoramidite (110 mL, 0.327 mmol) was added dropwise. After the addition, the solution stirred for 3 h at r.t.. The mixture was then cooled to -40 ° C and a solution of 70% 3-chloroperbenzoic acid (50 mg, 0.203 mmol) in CH₂Cl₂ (3 mL) was added. The mixture was stirred for 2 h 30 min, diluted with CH₂Cl₂ (40 mL), washed with sat. NaHSO₃ (2x50 mL), sat. NaHCO₃ (2x50 mL) and sat. NaCl (2x50 mL), dried over MgSO₄ and concentrated. Flash chromatography (hexane/AcOEt 4:1) gave compound **15** (68 mg, 0.061 mmol, 75%).

### 1'-D-6-O-(2'-azido-3'-dibenzyl-phosphate-2'-deoxy-α-D-glucopyranosyl)-3,4,5-tri-O-benzyl-myo-inositol (16)

To a solution of disaccharide **15** (30 mg, 0.027 mmol) in CH₂Cl₂ (3 mL), H₂O (0.06 mL, 3.330 mmol) and trifluoroacetic acid (249 mL, 3.243 mmol) were added, and the reaction stirred for 18 h at r.t. The mixture was then diluted with CH₂Cl₂ (25 mL), washed with sat. NaHCO₃ (2x25 mL), sat. NaCl (3x25 mL), dried over MgSO₄ and concentrated. Flash chromatography (hexane/AcOEt 1/3 to AcOEt 100% and finally AcOEt/MeOH 9/1) gave compound **16** (16 mg, 0.018 mmol, 67%).

### 1'-D-6-O-(2'-amino-2'-deoxy-3'-phosphate-α-D-glucopyranosyl)-myo-inositol (RGL1029)

To a suspension of disaccharide **16** (8 mg, 8.910 mmol) in a mixture MeOH/H₂O 4/1 (0.3 mL), 10% Pd/C (2.1 mg, 0.002 mmol) was added and stirred under hydrogen atmosphere at r.t. for 18 h. The MeOH was evaporated, the crude suspended in dest. H₂O, filtered through celite and the filtrate lyophilized to give **RGL1029** (3.6 mg, 8.545 mmol, 96%) as a white solid. ¹H-NMR (D₂O, 500 MHz): δ 5.30 (broad s, 1H, H_{1'}), 4.22 (broad s, 1H, H_{3'}), 3.97 (m, 1H, H_{5'}), 3.90 (broad s, 1H, H₂), 3.70 (m, 2H, H_{6'a}+H_{6'b}), 3.66 (broad d, 1H, H₁), 3.60 (broad t, *J*= 9.1 Hz, 1H, H₆), 3.54 (m, 1H, H_{4'}), 3.51 (t, *J=* 9.1 Hz, H, H₄), 3.41 (dd, *J*₁= 9.1 Hz, *J*₂=4.1 Hz, 1H, H₃), 3.31 (t, *J*=9.1 Hz, 1H, H₅), 3.25 (m, 1H, H_{2'}). ¹³C-NMR (D₂O, 500 MHz): d 97.20 (C_{1'}), 80.88 (C₆), 73.42 (C_{3'}), 72.8 (C₅), 72.82 (C₄), 72.76 (C₂), 72.0 (C_{5'}), 71.91 (C₁), 71.25 (C₃), 69.52 (C_{4'}), 60.25 (C_{6'a}+C_{6'b}), 54.59 (C_{2'}).

### Synthesis Of RGL 1134 (Scheme 6)

### 1'-D-6-O-(2'-azido-3'-O-benzyl-2'-deoxy-α-D-glucopyranosyl)-3,4,5-tri-O-benzyl-myo-inositol (2)

To a mixture of the disaccharide **1** (0.72 g, 0.75 mmol) in DCM (45 ml) was added water (0.82 ml, 0.82 g, 45.6 mmol) and trifluoroacetic acid (5.18 g, 3.5 mmol, 45.4 mmol) at room temperature for 1.25 h. The mixture was diluted with DCM (100 ml) and washed with NaHCO₃ (3 x 50 ml) and brine (1 x 50 ml). The solvent was dried (MgSO₄), filtered and concentrated *in vacuo* to yield a crude oil (0.84 g). The tetrol **2** (0.37 g, 67%) was isolated after flash chromatography (silica, 60→80→100% ethyl acetate/hexanes).

### 1'-D-6-O-(2'-azido-3'-O-benzyl-6'-O-DMT-2'-deoxy-α-D-glucopyranosyl)-3,4,5-tri-O-benzyl-myo-inositol (3)

The tetrol **2** (110 mg, 0.15 mmol) was co-evaporated with pyridine (2 x 1 ml) and dissolved in pyridine (2 ml). To the solution was added DMTCl (78 mg, 1.5 eq) and the reaction stirred for 16 h. The solvent was removed *in vacuo* and the residue dissolved in DCM (30 ml). The solution was washed with NaHCO₃ (sat, 2 x 20 ml), dried (MgSO₄), filtered and concentrated *in vacuo.* The residual pyridine was removed by co-evaporation with toluene. The product (100 mg, 97 µmol) was isolated by flash chromatography (silica, 10%→50% ethyl acetate/hexanes).

### 1'-D-6-O-(2'-azido-3'-O-benzyl-4'-O-phosphate-6'-O-DMT-2'-deoxy-α-D-glucopyranosyl)-3,4,5-tri-O-benzyl-myo-inositol-1,2-cyclic phosphate, sodium salt (4)

Methyldichlorophosphate (30 µl, 0.29 mmol, 3 eq) was added to pyridine (630 µl) with stirring under an atmosphere of nitrogen at room temperature. After 30 min the triol **3** (100 mg, 0.10 mmol) in pyridine (100 µl) was added and the reaction stirred for a further 4 h. The reaction was diluted with DCM (10 ml) and quenched by addition of NaHCO₃ solution. The resulting emulsion was separated by the addition of brine and the aqueous phase extracted with DCM (1 x 10 ml). The combined organic solvents were dried (Na₂SO₄), filtered and concentrated *in vacuo* to yield the required product **4** (17 mg, 13.8 µmol, 14%, R_{f} 0.04 20% ethyl acetate/hexanes).

### 1'-D-6-O-(2'-azido-3'-O-benzyl-4'-O-phosphate-2'-deoxy-α-D-glucopyranosyl)-3,4,5-tri-O-benzyl-myo-inositol-1,2-cyclic phosphate, sodium salt (5)

To a stirred solution of compound **4** (17 mg, 13.4 µmol) in DCM (2 ml) was added a solution of TFA in DCM (2 %, 2 ml) at 0°C. TLC indicated the reaction proceeded to completion in *ca.* 1 min. The reaction was quenched by addition of NaHCO₃ (sat. 5 ml), diluted with DCM (10 ml). The phases were separated and the organic phase dried (Na₂SO₄), filtered and concentrated *in vacuo.* The residue was purified by column chromatography (silica, 1:1 ethyl acetate/hexanes→ethyl acetate) to yield the product as a colourless solid (10 mg, 10.8 µmol, 81%). ¹H-NMR (250 MHz), δ (ppm): 7.25 (m, 20 H); 5.3 (d, 1 H, H-1'); 5.0 (d, 1 H); 4.7 (m); 7.6 (*bs,* 1 H); 3.9 (m, 2 H); 3.64 (m); 3.3 (m); 2.62 (s, 1 H); 2.22 (d, 1 H).

### 1'-D-6-O-(2'-amino-3'-O-benzyl-4'-O-phosphate-2'-deoxy-α-D-glucopyranosyl)-3,4,5-tri-O-benzyl-myo-inositol-1,2-cyclic phosphate, sodium salt (6, RGL 1134)

Pd/C (10%, 5 mg) was added to a solution of compound **5** (10 mg, 10.8 µmol) in methanol/THF/H₂O (1:1:1, 3 ml) and stirred at room temperature overnight under an atmosphere of hydrogen. The mixture was filtered through celite and the celite washed with methanol (2 x 5 ml) and water (2 x 5 ml). The solution was concentrated *in vacuo* to yield a colourless solid. Residual celite was removed by filtration through cotton wool and the crude product **RGL 1134** (12 mg) was isolated after evaporation *in vacuo.* ¹H-NMR (250 MHz), δ(ppm): 7.4 (m, 20 H); 5.38 (d, 1 H, H-1'); 5.0 (d, 1 H); 4.8 (m); 4.18 (*b*s, 1 H); 3.9 (m, 2 H); 3.8 (*b*d, 1 H); 3.6 (m, 2 H); 3.22 (m, 4 H); 2.65 (m, 1 H).

### Synthesis of RGL1135 (Scheme 7)

### 1'-D-6-O-(2'-azido-3'-D-benzyl-4',6'-di-O-cyclic phosphate-2'-deoxy-α-D-glucopyranosyl)-3,4,5-tri-O-benzyl-myo-inositol-1,2-cyclic phosphate, triethylammonium salt (2)

Methyldichlorophosphate (30 µl, 0.29 mmol, 3 eq) was added to pyridine (630 µl) with stirring under an atmosphere of nitrogen at room temperature. After 30 min compound **1** (100 mg, 0.14 mmol) in pyridine (100 µl) was added and the reaction stirred for a further 4 h. The reaction was diluted with DCM (10 ml) and quenched by addition of NaHCO₃ solution. The resulting emulsion was separated by the addition of brine and the aqueous phase extracted with DCM (1 x 10 ml). The combined organic solvents were dried (Na₂SO₄), filtered and concentrated *in vacuo.* The crude material was purified by preparative TLC (methanol/DCM/Et₃N 10:90:1) to yield compound **2** (11 mg, 7.5%).

### 1'-D-6-O-(2'-amino-3'-O-benzyl-4',6'-di-O-cyclic phosphate-2'-deoxy-α-D-glucopyranosyl)-3,4,5-tri-O-benzyl-myo-inositol-1,2-cyclic phosphate, triethylammonium salt (3, RGL 1135)

Compound **2** (11 mg, 10.4 µmol) in THF/ethanol/water (1:1:1,3 ml) was stirred at room temperature for 22 h under an atmosphere of hydrogen. The mixture was filtered through celite and washed with methanol. The solvent was removed *in vacuo* to yield **RGL 1135** (8 mg, 71%). ¹H-NMR (250 MHz), δ (ppm): 7.25 (m, 20 H); 5.6 (d, 1 H, H-1'); 5.0 (d, 1 H); 4.8 (m); 4.4 (m); 4.2 (m); 4.0 (m); 3.7 (m); 3.45 (m, 1 H); 3.25 (dd, 1H); 3.1 (c); 2.1 (s, 1H); 1.3 (t).

### Synthesis of RGL 1133 (Scheme 8)

### 1'-D-6-O-(2'-azido-3'-O-benzyl-4',6'-di-O-sulphate-2'-deoxy-α-D-glucopyranosyl)-3,4,5-tri-O-benzyl-myo-inositol-1,2-di-O-sulphate, sodium salt (2).

A mixture of the tetrol **1** (88 mg, 0.12 mmol), sulfur trioxide-trimethylamine complex (0.32 g, 2.30 mmol, 20 eq) in DMF was heated for 16 h at 50°C. The DMF was removed *in vacuo* and the residue dissolved in MeOH/water (9:1,5 ml). Portions (1 ml) were taken for prep. TLC (R_{f} 0.2, 4:1 EtOH/0.88 ammonia solution). Each 1 ml portion yielded *ca.* 15 mg (11 % per pTLC). The ammonium salts were dissolved in MeOH/water (9:1) and passed through Dowex (Na⁺) to give a quantitative return of the sodium salt after concentration *in vacuo,* re-dissolution in water and lyophilisation. ¹H-NMR (250 MHz), δ(ppm): 7.35 (m 20 H); 5.85 (d, 1 H), 5.55 (s, 1 H); 5.15 (d, 1 H), 4.8 (m), 4.25 (m); 3.95 (c, 2 H); 3.48 (m, 3 H); 3.25 (m); 2.95. IR (cm⁻¹): 3460.44; 2111.27; 1633.18; 1496.32; 1453.97; 1213.66; 1123.57; 1047.01; 1016.31; 922.85; 802.42; 747.37; 696.33; 582.86

### 1'-D-6-O-(2'-amino-3'-O-benzyl-4',6'-di-O-sulphate-2'-deoxy-α-D-glucopyranosyl)-3,4,5-tri-O-benzyl-myo-inositol-1,2-di-O-sulphate, sodium salt (3)

A mixture of the tetrasulfate **2** (17 mg, 15 µmol), Pd/C (10%, 13 mg), ammonium acetate (5 mg) in THF/ethanol/water (1:1:1,3 ml) was stirred at room temperature for 22 h. The mixture was filtered through celite and the celite washed with methanol. The solvent was removed *in vacuo* and the residue dissolved in water. Compound **3**, **RGL 1133** (14 mg, 84 %) was isolated after freeze drying. ¹H-NMR (250 MHz), δ (ppm): 7.4, (m, 20 H); 5.5, (s, 1 H); 5.18 (s, 1 H); 4.8 (m, 8 H), 4.1 (m, 6 H); 3.6 (m, 7 H). IR (cm⁻¹): 2924.62; 1573.89; 1496.74; 1453.79; 1214.25; 1130.12; 1048.06; 996.84; 925.29; 802.77; 748.57; 696.19; 579.54.

### Synthesis of RGL 1130 (Scheme 9)

### 1'-D-6-O-(2'-azido-3',6'-di-O-benzyl-2'-deoacy-α-D-glucopyranosyl)-1,2-O-(L-1,7,7-trimethyl[2.2.1]-bicyclohept-2-ylidene)-3,4,5-tri-O-benzyl-myo-inositol and 1'-D-6-O-(2'-azido-3',4'-di-O-benzyl-2'-deoxy-α-D-glucopyranosyl)-1,2-O-(L-1,7,7-trimethyl[2.2.1]-bicyclohept-2-ylidene)-3,4,5-tri-O-benzyl-myo-inositol (2)

Compound **1** (150 mg, 0.16 mmol) was dissolved in dry THF (3.75 ml) and sodium cyanoborohydride (2.07 ml, 1 M in THF) was added. The mixture was stirred at room temperature for 15 min and hydrogen chloride (*ca.* 1.8 ml, 1 M in diethyl ether) added until gas evolution ceased. The turbid mixture was stirred for a further 1.5 h. The mixture was diluted with DCM (10 ml) and washed with NaHCO₃ (sat, 2 x 25 ml). The aqueous phase was extracted with DCM (2 x 20 ml). The combined organic extracts were dried (MgSO₄), filtered and concentrated *in vacuo.* The resulting yellow material was purified by column chromatography (silica, 15%→20% ethyl acetate/hexanes) yielding the desired product (110 mg, 73%, 1:1 mixture of 4' and 6' ethers).

### 1'-D-6-O-(2'-azido-3',6'-di-O-benzyl-4'-O-(dibenzyl)phosphate-2'-deoxy-α-D-glucopyranosyl)-1,2-O-(L-1,7,7-trimethyl[2.2.1]-bicyclohept-2-ylidene)-3,4,5-triO-benzyl-myo-inositol and 1'-D-6-O-(2'-azido-3',4'-di-O-benzyl-6'-O-(dibenzyl)phosphate 2'-deoxy-α-D-glucopyranosyl)-1,2-O-(L-1,7,7-trimethyl[2.2.1]-bicyclohept-2-ylidene)-3,4,5-tri-O-benzyl-myo-inositol (3)

To a solution of the disaccharides **2** (100 mg, 0.1 mmol) and tetrazole (38 mg, 0.55 mol) in dry DCM (10 ml) at 0°C was added dibenzyl diisopropylphosphramidite (140µl). After 2.5 h, the mixture was cooled to -40°C and *m*CPBA (60 mg) in DCM (4 ml) was added. The reaction mixture was warmed to room temperature and stirred for 2.5 h. The mixture was diluted with DCM (20 ml), washed with sodium metabisulfite (2 x 10 ml), NaHCO₃ (sat. 2 x 10 ml) and brine (2 x 10 ml). The solution was dried (MgSO₄) and concentrated *in vacuo.* The mixture of products (100 mg, 78%) was isolated after column chromatography (silica, 20% ethyl acetate/hexanes).

### 1'-D-6-O-(2'-azido-3',6'-di-O-benzyl-4'-O-(dibenzyl)phosphate-2'-deoxy-α-D-glucopyranosyl)-3,4,5-tri-O-benzyl-myo-inositol and 1'-D-6-O-(2'-azido-3',4'-di-O-benzyl-6'-O-(dibenzyl)phosphate 2'-deoxy-α-D-glucopyranosyl)-3,4,5-tri-O-benzyl-myo-inositol (4)

To a mixture of Compounds **3** (110 mg, 89.6 µmol) in DCM (45 ml) was added water (approx. 1 ml) and TFA (3.5 ml) at room temperature and kept stirring for 2 h. The mixture was diluted with DCM (100 ml) and washed with NaHCO₃ (3 x 50 ml) and brine (50 ml). The solvent was dried (MgSO₄), filtered and concentrated to yield compounds **4** (80 mg, 82%), which were isolated after column chromatography (silica, 50%→60% ethyl acetate/hexanes).

### 1'-D-6-O-(2'-amino-4'-O-phosphate-2'-deoxy-α-D-glucopyranosyl)-myo-inositol-1,2-cyclic phosphate and 1'-D-6-O-(2'-amino-6'-O-phosphate-2'-deoxy-α-D-glucopyranosyl)-myo-inositol-1,2-cyclic phosphate (5, RGL 1130)

Dichloromethylphosphate (40 µl) was added to pyridine (700 µl) and stirred for 30 min. Compounds 4 (80 mg, 73.1 µmol) were dissolved in a minimum of pyridine and added to the dichloromethylphosphate solution and stirred for 2.5 h. NaHCO₃ (sat. 2 ml) was added and the solvent removed *in vacuo.* The residue was taken up in water (10 ml) and acidified (pH 1) with dilute HCl. The aqueous solution was extracted with ethyl acetate (2 x 50 ml), dried (MgSO₄) and concentrated under reduced pressure. The oil was dissolved in THF/EtOH/water (1:1:1,15 ml) and the resulting solution was degassed and flushed with nitrogen. Pd/C (10 %, 150 mg) and ammonium acetate (40 mg) were added and the reaction placed under an atmosphere of hydrogen. The mixture was stirred for 24 h and the reaction mixture filtered through celite and the celite was washed with water and ethanol. The solvent was removed *in vacuo* and the residue co-evaporated with ethanol (2 x) and toluene (3 x) to give compound **5, RGL 1130** as a crude solid (30 mg). ¹H-NMR (500 MHz), δ (ppm): 5.38 (*bs*); 4.7 (m); 4.5 (dt); 4.17-3.17 (m); 2.7 (s); 2.4-2.28 (m); 2.23-2.03 (m); 1.13-1.19 (m).

### Assay Data

### PDH activation:

| | **100µM** |
|---|---|
| RGL1023 | 14% |
| RGL1027 | 132% |
| RGL1029 | 361% |

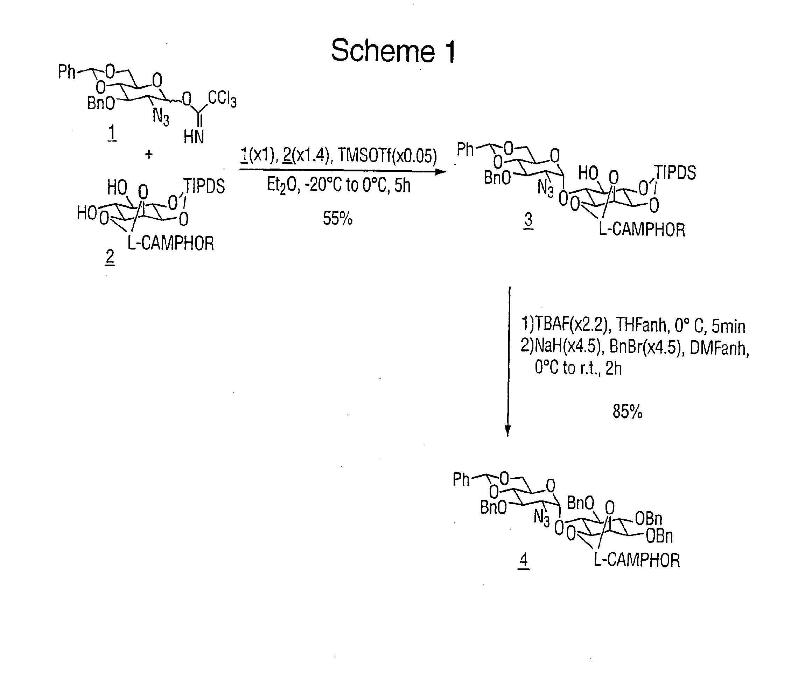

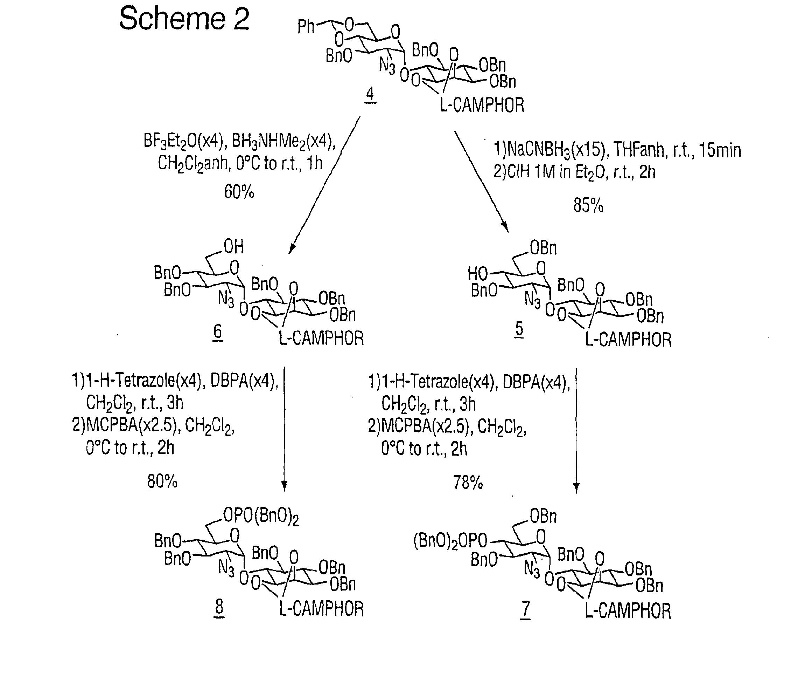

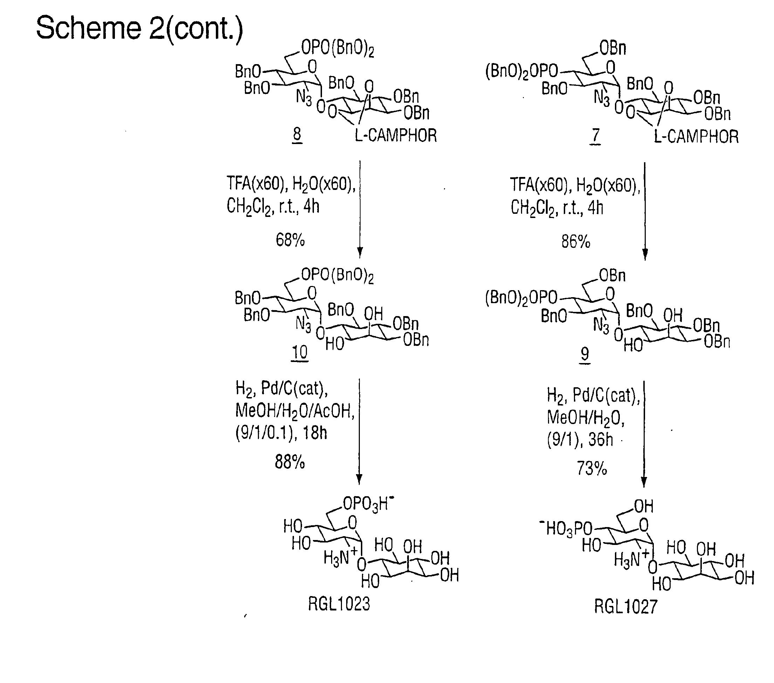

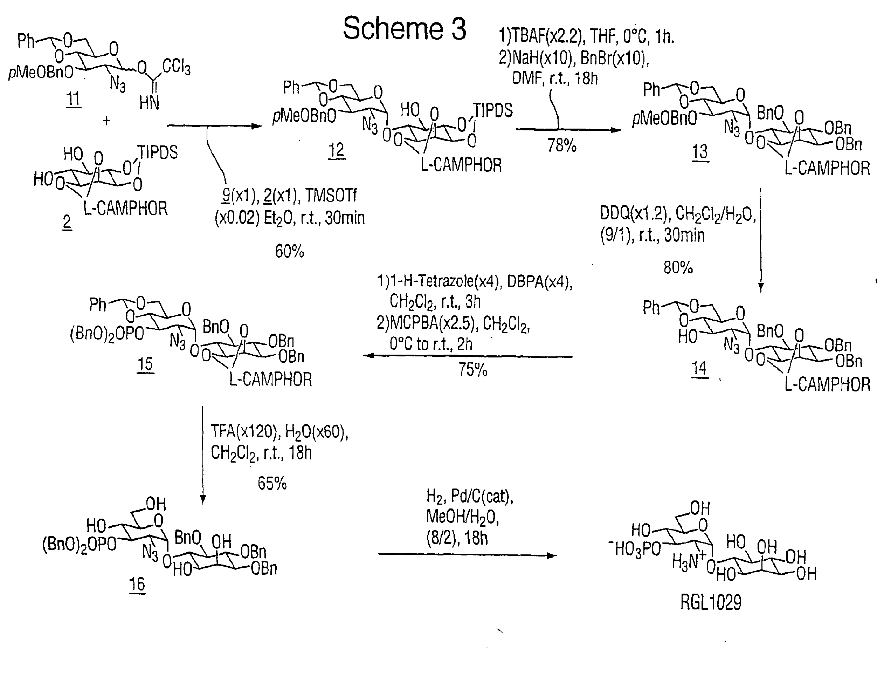

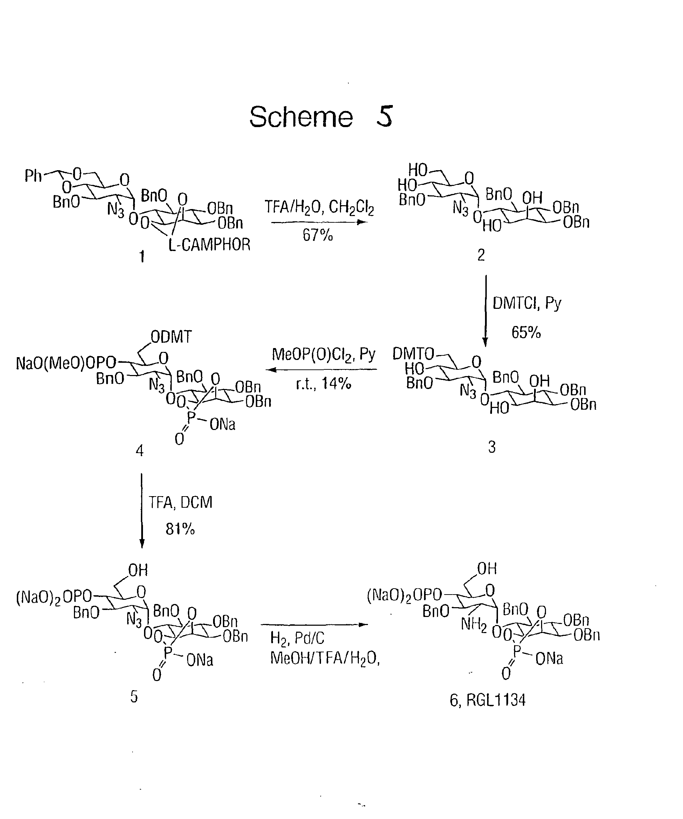

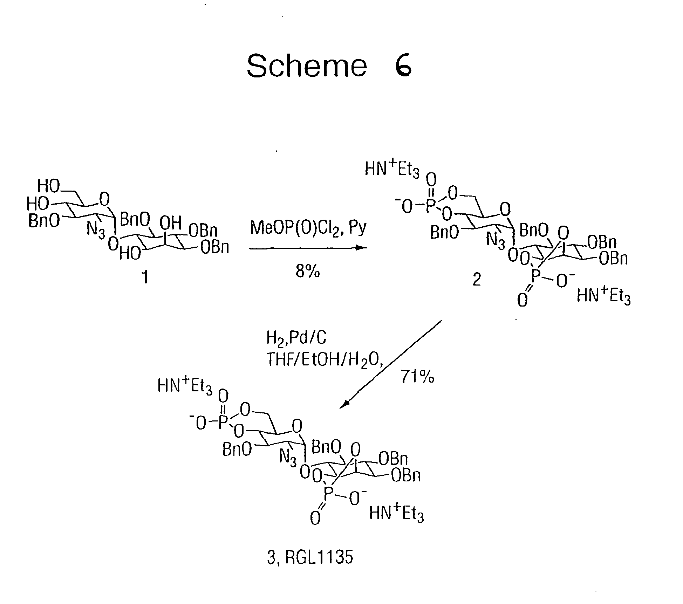

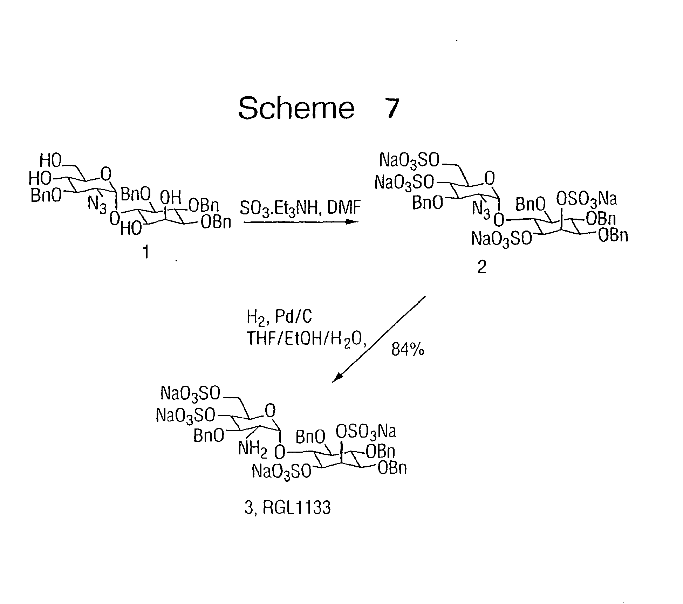

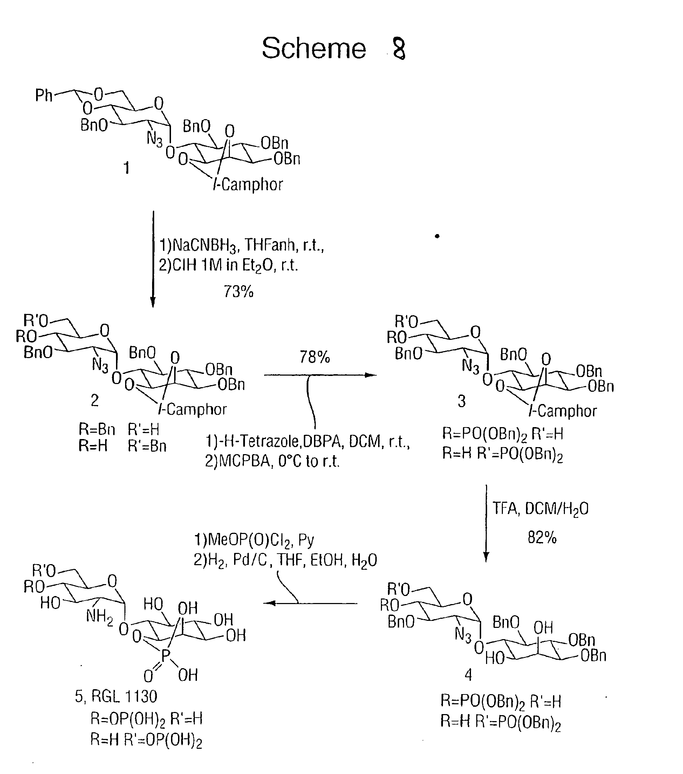

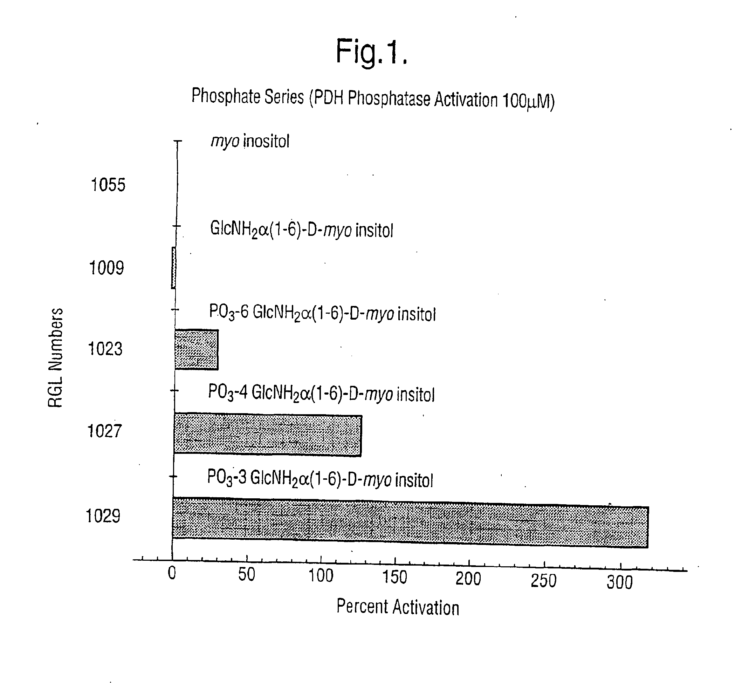

These results are represented schematically in Figure 1, which compares the results obtained for the first three compounds of the invention with *myo*-inositol.

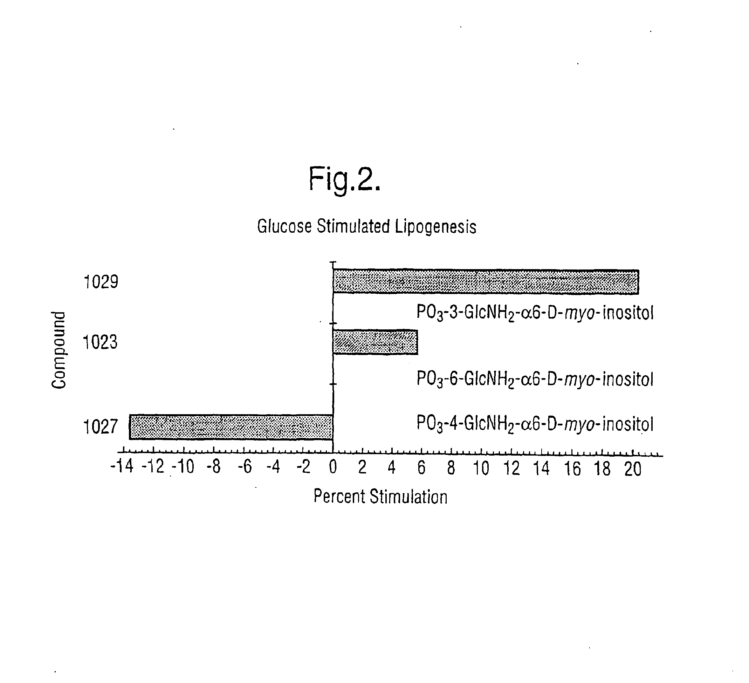

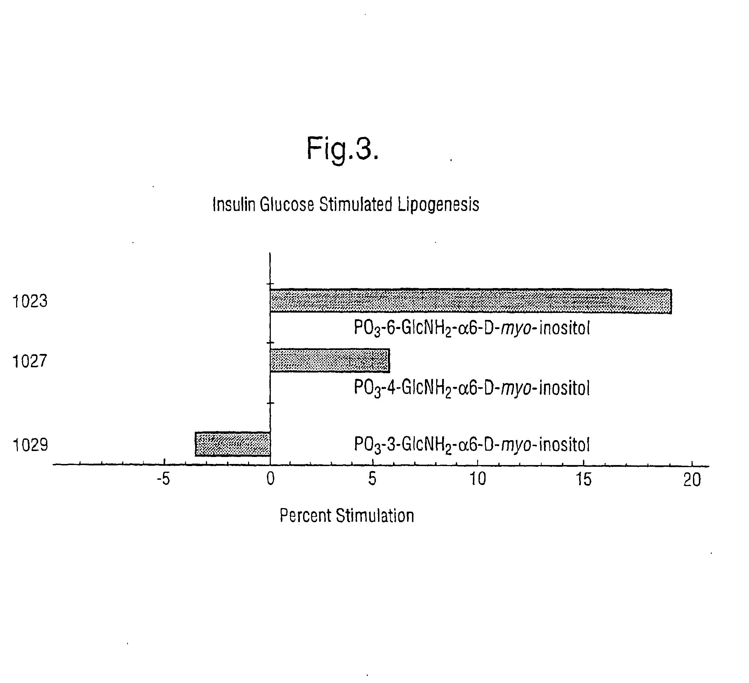

Figures 2 and 3 report the effect of compounds RGL1023, 1027 and 1029 on glucose and insulin stimulated lipogenesis. These results show that compounds RGL1029 and 1023 stimulate lipogenesis in both assays while RGL1027 inhibits lipogenesis.

### PKA inhibition:

| | **0.1µM** | **10µM** |
|---|---|---|
| RGL1027 | 18% | 10% |
| RGL1029 | 59% | 33% |

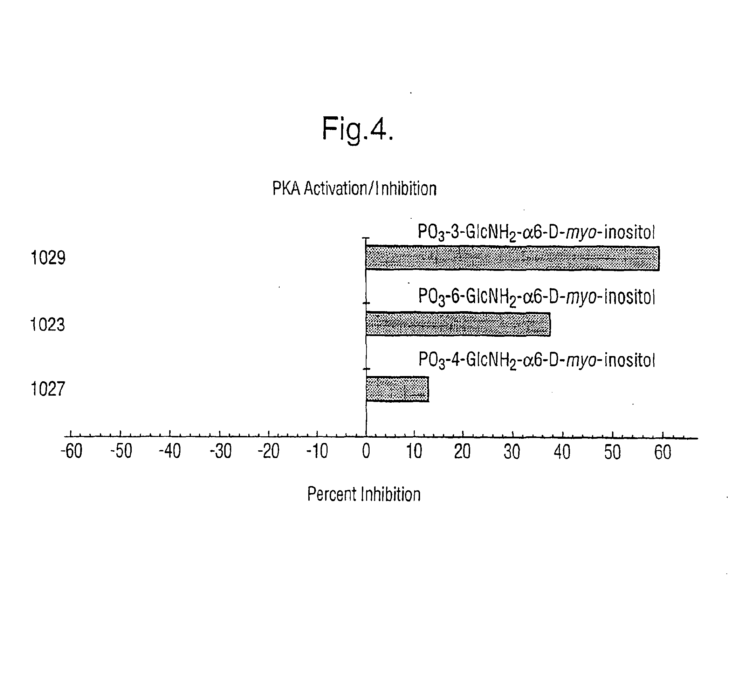

These results and those for compound RGL1023 are reported in Figure 4.

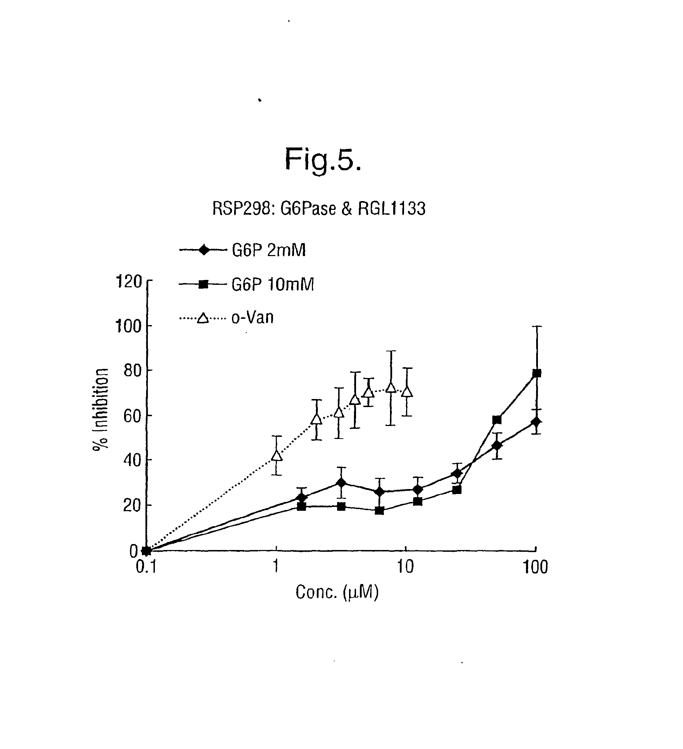

Figure 5 shows the result of an assay measuring the effect of compound RGL1133 in inhibiting glucose 6-phosphatase, as compared to an known inhibitor, sodium O-vanadate. The results show that RGL1133 is a good inhibitor of G6Pase activity and would be useful for decreasing hepatic glucose output in diabetic subjects. This suggests that this compound would be useful in the treatment of both type I and type II diabetes.

### References:

[1] (a) Varela-Nieto et al, Comp. Biochem. Physiol.,. 115B:223-241, 1996; (b) Strälfors, Bioassays, 19:327-335, 1997; (c) Field, Glycobiology, 7:161-168, 1997; (d) Jones & Varela-Nieto, Int. J. Biochem. Cell Biol., 30:313-326, 1998.
[2] Mato et al, Biochem. Biophys. Res. Commun., 146:746-770, 1987.
[3] Lamer et al, Biochem. Biophys. Res. Commun., 151:1416-1426, 1998.
[4] Caro et al, Biochem. Mol. Med, 61:214-228, 1997.
[5] For reviews on the synthesis of these structures see: (a) Gigg & Gigg in Glycopeptides and Related Compounds, Large & Warren, Eds., Marcel Dekker, New York, 1997, pp 327-392; (b) Khiar & Martin-Lomas in Carbohydrate Mimics. Concepts and Methods, Chapleur Ed Wiley VCH, 1998, pp 443-462; (c) Dietrich et al, Chem. Eur. J., 5:320-336, 1999.
[6] Jaramillo et al, J. Org. Chem., 59, 3135-3141, 1994.
[7] Corey & Venkateswarlu, J. Am. Chem. Soc., 94:6190, 1974.
[8] Ley et al, Angew.Chem. Int. Ed. Engl.,33:2290-2292, 1994.
[9] Kinzi & Schmidt, Liebigs Ann. Chem., 1537-1545, 1985.
[10] Vasella et al, Helv. Chim. Acta., 74:2073-2077, 1991.
[11] Schmidt & Kinzi, Adv. Carbohydy. Chem. Biochem., 50:21-123, 1994.
[12] Once et al, Chem. Eur. J., 3:431-440, 1997.
[13] Rademacher et al, Brazilian J. Med. Biol. Res., 27:327-341, 1994.
[14] Caro et al, Biochem. Molec. Med., 61:214-228, 1997.
[15] Kunjara et al, In: Biopolymers and Bioproducts: Structure, Function and Applications, Ed Svati et al, 301-305, 1995.
[16] Zapata et al, Carbohydrate Res., 264:21-31, 1994.
[17] Dietrich et al, Chem. Eur. J.,5:320-336, 1999.
[18] Baeschlin et al, Chem. Eur. J., 6(1):172-186,2000.
WO98/11116 and WO98/11117 (Rademacher Group Limited).
WO98/11435 and WO98/10791 (Rademacher Group Limited).
WO99/38516 (Rademacher Group Limited).

## Claims

1. A compound represented by the general formula:
X-α1,6-D-myo-inositol
wherein:
X represents a sugar residue;
the sugar residue is substituted with one or more negatively charged groups selected from phosphate-O-P(O)(OH); thiophosphate -O-P(S)(OH)₂; phosphate -O-P(O)OHR; thiophosphonate -O-P(S) OHR; -O-P(S)(OH)(SH); cyclic phosphate sulphate OSO₃H, -O-S(O)(OH) and carboxylic acid,
the sugar residue is optionally further substituted with between one and three groups, and the myo-inositol is unsubstituted or is substituted with between one and five groups, the group or groups independently selected from:
(a) a phosphoryl group which is phosphate -O-P(O)(OH)₂; thiophosphate -OP(S)(OH)₂; phosphate esters -O-P(O)(OR)₂; thiophosphate esters -OP(S)(OR)₂; phosphonate -O-P(O)OHR; thiophosphonate -O-P(S)OHR; substituted phosphonate -O-P(O)OR₁R₂; substituted thiophosphonate -O-P(S)OR₁R₂; -O-P(S)(OH)(SH); or cyclic phosphate;
(b) a phosphorus containing compounds which is phosphoramidite -O-P(OR)-NR₁R₂ or phosphoramidate -O-P(O)(OR)-NR₁R₂;
(c) a sulphur group which is -O-S(O)(OH), -SH, -SR, -S(-O)-R, -S(O)₂R, RO-S(O)₂⁻, -O-SO₂NH₂, -O-SO₂R₁R₂ or sulphamide -NHSO₂NH₂;
(d) an amino group which is -NHR, -NR₁R₂, -NHAc, -NHCOR, -NH-O-COR,-NHSO₃⁻, -NHSO₂R or -N(SO₂R)₂, and/or an amidino group which is -NH-C(=NH)NH₂ and/or an ureido group which is -NH-CO-NR₁R₂ or a thiouriedo group which is -NH-C(S)-NH₂;
(e) a hydroxy group or substituted hydroxy group which is -OR₃, where R₃ is C₁-₁₀ unsubstituted or substituted alkyl, e.g. CHF₂ or CF₃, alkoxyalkyl, aryloxyalkyl, cycloalkyl, alkenyl (unsubstituted alkyl), alkylene (C₃₋₇ cycloalkyl), -OCOR, aryl, heteroaryl, acetal, or where two hydroxyl group are joined as a ketal;
(f) a halogen substituents which is fluorine or chlorine;
(g) hydrogen, to provide a deoxy sugar;
wherein R, R₁ and R₂ are independently hydrogen or C₁₋₁₀ unsubstituted or substituted alkyl or aryl;
or a salt, coordination complex with metal ions, ester, free acid or base or, hydrate thereof, or a glycolipid derivative in which a lipid moiety clearable by a phospholipase enzyme is a substituent of the sugar residue or the cyclitol.

2. The compound of claim 1, wherein the sugar residue is a hexose or a pentose, or substituted forms thereof.

3. The compound of claim 2, wherein the sugar residue is a hexose selected from the group consisting of glucose, galactose or mannose.

4. The compound of claim 2, wherein the sugar residue is a hexosamine.

5. The compound of claim 4, wherein the hexosamine is galactosamine or glucosamine.

6. The compound of any one of the preceding claims which is selected from the group consisting of:
RGL1023 *O*-(2'-amino-2'-deoxy-6'-phosphate-D-glucopyranosyl)-α(1,6)-D-*myo-*inositol;
RGL1027 *O*-(2'-amino-2'-deoxy-4'-phosphate-D-glucopyranosyl)-α(1,6)-D-*myo-*inositol;
RGL1029 *O*-(2'-amino-2'-deoxy-3'-phosphate-D-glucopyranosyl)-α(1,6)*-*D*-myo-*inositol;
RGL1134 1'-D-6-*O*-(2'-amino-3'-*O*-benzyl-4'-*O*-phosphate-2'-deoxy-α-D-glucopyranosyl)-3,4,5-tri-*O*-benzyl-*myo*-inositol-1,2-cyclic phosphate;
RGL1133 1'-D-6-*O*-(2'-amino-3'-*O*-benzyl-4',6'-di-*O*-sulphate-2'-deoxy-α-D-glucopyranosyl)-3,4,5-tri-O-benzyl-myo-inositol-1,2-di-O-sulphate;
RGL1135 1'-D-6-O-(2'-amino-3'-O-benzyl-4',6'-di-O-cyclic phosphate-2'-deoxy-α-D-glucopyranosyl)-3,4,5-tri-O-benzyl-myo-inositol-1,2-cyclic phosphate;
RGL1130 1'-D-6-*O*-(2'-amino-4'-*O*-phosphate-2'-deoxy-α-D-glucopyranosyl)-*myo*-inositol-1,2-cyclic phosphate and 1'-D-6-*O*-(2'-amino-6'-*O-*phosphate-2'-deoxy-α -D-glucopyranosyl)-*myo*-inositol-1,2-cyclic phosphate;
or a salt coordination complex with metal ions, ester free acid or base, or hydrate thereof, or a glycolipid derivative in which a lipid moiety cleavable by a phospholipase enzyme is a substituent of the sugar residue or cyclitol.

7. A composition comprising a compound of any one of the preceding claims in combination with a pharmaceutically acceptable carrier.

8. A compound of any one of claims 1 to 6 for use in a method of medical treatment.

9. Use of a compound of any one of claims 1 to 6 for the preparation of a medicament for the treatment of a condition ameliorated by the administration of an inositol phosphoglycan (IPG) second messenger or an IPG antagonist.

## Patentansprüche

1. Verbindung der allgemeinen Formel:
X-α1,6-D-myo-Inosit
worin:
X für einen Zuckerrest steht;
der Zuckerrest mit einer oder mehreren negativ geladenen Gruppen substituiert ist, die aus Phosphat -O-P(O)(OH)₂; Thiophosphat -O-P(S)(OH)₂; Phosphonat -O-P(O)OHR; Thiophosphonat -O-P(S)OHR; -O-P(S)(OH)(SH); zyklischem Phosphat; Sulfat -OSO₃H; -O-S(O)(OH) und Carbonsäure ausgewählt sind;
der Zuckerrest gegebenenfalls mit zwischen einer und drei Gruppen weiter substituiert ist und der myo-Inosit unsubstituiert oder mit zwischen einer und fünf Gruppen substituiert ist, wobei die Gruppen unabhängig voneinander ausgewählt sind aus:
(a) einer Phosphorylgruppe, die Phosphat -O-P(O)(OH)₂; Thiophosphat -O-P(S)(OH)₂; ein Phosphatester -O-P(O)(OR)₂; ein Thiophosphatester -O-P(S)(OR)₂; Phosphonat -O-P(O)OHR; Thiophosphonat -O-P(S)OHR; substituiertes Phosphonat -O-P(O)OR₁R₂; substituiertes Thiophosphonat -O-P(S)OR₁R₂; -O-P(S)(OH)(SH); oder zyklisches Phosphat ist;
(b) einer phosphorhältigen Verbindung, die Phosphoramidit -O-P(OR)-NR₁R₂ oder Phosphoramidat -O-P(O)(OR)-NR₁R₂ ist;
(c) einer Schwefelgruppe, die -O-S(O)(OH), -SH, -SR, -S(→O)-R, -S(O)₂R, RO-S(O)₂⁻, -O-SO₂NH₂, -O-SO₂R₁R₂ oder Sulfamid -NHSO₂NH₂ ist;
(d) einer Aminogruppe, die -NHR, -NR₁R₂, -NHAc, -NHCOR, -NH-O-COR, -NHSO₃⁻, -NHSO₂R oder -N(SO₂R)₂ ist, und/oder einer Amidinogruppe, die -NH-C(=NH)NH₂ ist, und/oder einer Ureidogruppe, die -NH-CO-NR₁R₂ ist, oder einer Thioureidogruppe, die -NH-C(S)-NH₂ ist;
(e) einer Hydroxygruppe oder einer substituierten Hydroxygruppe, die -OR₃ ist, worin R₃ unsubstituiertes oder substituiertes C₁₋₁₀-Alkyl, z.B. CHF₂ oder CF₃, -Alkoxyalkyl, -Aryloxyalkyl, -Cycloalkyl, -Alkenyl (unsubstituiertes Alkyl), -Alkylen (C₃₋₇-Cycloalkyl), -OCOR, -Aryl, -Heteroaryl oder -Acetal ist oder worin zwei Hydroxylgruppen als Ketal verbunden sind;
(f) einem Halogensubstituenten, der Fluor oder Chlor ist;
(g) Wasserstoff, um einen Desoxyzucker bereitzustellen;
worin R, R₁ und R₂ unabhängig voneinander Wasserstoff oder unsubstituiertes oder substituiertes C₁₋₁₀-Alkyl oder -Aryl sind;
oder ein Salz, ein Koordinationskomplex mit Metallionen, ein Ester, eine freie Säure oder Base oder ein Hydrat davon oder ein Glykolipid-Derivat, in dem eine von einem Phospholipase-Enzym spaltbare Lipidgruppierung ein Substituent des Zuckerrests oder des Cyclits ist.

2. Verbindung nach Anspruch 1, worin der Zuckerrest eine Hexose oder eine Pentose oder eine substituierte Form davon ist.

3. Verbindung nach Anspruch 2, worin der Zuckerrest eine Hexose ist, die aus der aus Glucose, Galactose und Mannose bestehenden Gruppe ausgewählt ist.

4. Verbindung nach Anspruch 2, worin der Zuckerrest ein Hexosamin ist.

5. Verbindung nach Anspruch 4, worin das Hexosamin Galactosamin oder Glucosamin ist.

6. Verbindung nach einem der vorangegangenen Ansprüche, die aus der aus Folgendem bestehenden Gruppe ausgewählt ist:
RGL1023 O-(2'-Amino-2'-desoxy-6'-phosphat-D-glucopyranosyl)-α(1,6)-D-myo-inosit;
RGL1027 O-(2'-Amino-2'-desoxy-4'-phosphat-D-glucopyranosyl)-a(1,6)-D-myo-inosit;
RGL1029 O-(2'-Amino-2'-desoxy-3'-phosphat-D-glucopyranosyl)-α(1,6)-D-myo-inosit;
RGL1134 1'-D-6-O-(2'-Amino-3'-O-benzyl-4'-O-phosphat-2'-desoxy-α-D-glucopyranosyl)-3,4,5-tri-O-benzyl-myo-inosit-1,2-(cyclo)phosphat;
RG L 1133 1'-D-6-O-(2'-Amino-3'-O-benzyl-4',6'-di-O-sulfat-2'-desoxy-α-D-glucopyranosyl)-3,4,5-tri-O-benzyl-myo-inosit-1,2-di-O-sulfat;
RG L 1135 1'-D-6-O-(2'-Amino-3'-O-benzyl-4',6'-di-O-(cyclo)phosphat-2'-desoxy-α-D-glucopyranosyl)-3,4,5-tri-O-benzyl-myo-inosit-1,2-(cyclo)phosphat;
RGL1130 1'-D-6-O-(2'-Amino-4'-O-phosphat-2'-desoxy-α-D-glucopyranosyl)-myo-inosit-1,2-(cyclo)phosphat und 1'-D-6-O-(2'-Amino-6'-O-phosphat-2'-desoxy-α-D-glucopyranosyl)-myo-inosit-1, 2-cyclophosphat;
oder ein Salz, ein Koordinationskomplex mit Metallionen, ein Ester, eine freie Säure oder Base oder ein Hydrat davon oder ein Glykolipidderivat, in dem eine von einem Phospholipase-Enzym spaltbare Lipidgruppierung ein Substituent des Zuckerrests oder Cyclits ist.

7. Zusammensetzung, die eine Verbindung nach einem der vorangegangenen Ansprüche in Kombination mit einem pharmazeutisch annehmbaren Träger umfasst.

8. Verbindung nach einem der Ansprüche 1 bis 6 zur Verwendung in einem medizinischen Behandlungsverfahren.

9. Verwendung einer Verbindung nach einem der Ansprüche 1 bis 6 zur Herstellung eines Medikaments zur Behandlung eines Leidens, das durch Verabreichung eines zweiten Inositphosphoglykan- (IPG-) Messengers oder eines IPG-Antagonisten gelindert wird.

## Revendications

1. Composé représenté par la formule générale :
X-α1,6-myo-inositol
dans laquelle :
X représente un résidu de sucre ;
le résidu de sucre est substitué par un ou plusieurs groupes chargés négativement choisis parmi un phosphate -O-P-(O)(OH) ; un thiophosphate -O-P(S)(OH)₂ ; un phosphonate - O-P(O)OHR ; un thiophosphonate -O-P(S)OHR' ; -O-P(S)(OH)(SH) ; un phosphate cyclique ; un sulfate OSO₃H, -OS(O)(OH) et un acide carboxylique ;
le résidu de sucre est facultativement davantage substitué par un à trois groupes, et le myo-inositol est non substitué ou est substitué par un à cinq groupes, le groupe ou les groupes étant indépendamment choisis parmi :
(a) un groupe phosphoryle, qui est un phosphate -O-P-(O)(OH)₂ ; un thiophosphate -O-P(S)(OH)₂ ; des esters de phosphate -O-P(O)(OR)₂.; des esters de thiophosphate -O-P(S)(OR)₂; un phosphonate -O-P (O) OHR ; un thiophosphonate -O-P(S)OHR ; un phosphonate substitué -O-P(O)OR₁R₂ ; un thiophosphonate substitué -O-P (S) OR₁R₂ ; -O-P(S)(OH)(SH) ; ou un phosphate cyclique ;
(b) un composé contenant du phosphore qui est un phosphoramidite -O-P(OR)-NR₁R₂ ou un phosphoramidate -O-P(O)(OR)-NR₁R₂ ;
(c) un groupe soufré qui est -O-S(O)(OH), -SH, -SR, -S(-O) -R, -S(O)₂R, RO-S(O)₂⁻, -O-SO₂NH₂, -O-SO₂R₁R₂ ou un sulfamide -NHSO₂NH₂ ;
(d) un groupe amino qui est -NHR, -NR₁R₂, -NHAc, -NHCOR, -NH-O-COR, -NHSO₃⁻, -NHSO₂R ou -N(SO₂R)₂, et/ou un groupe amidino qui est -NH-C(=NH)NH₂ et/ou un groupe uréido qui est -NH-CO-NR₁R₂ ou un groupe thiouréido qui est -NH-C(S)-NH₂ ;
(e) un groupe hydroxy ou un groupe hydroxy substitué qui est -OR₃, où R₃ est un groupe alkyle en C_{1 à 10} non substitué ou substitué, par exemple CHF₂ ou CF₃, alcoxyalkyle, aryloxyalkyle, cycloalkyle, alcényle (alkyle non substitué), alkylène (cycloalkyle en C_{3 à 7}), -OCOR, aryle, hétéroaryle, acétal, ou bien où deux groupes hydroxyle sont joints sous forme de cétal ;
(f) un substituant halogène, qui est le fluor ou le chlore ;
(g) un atome d'hydrogène, pour former un désoxyose ;
dans lesquels R, R₁ et R₂ sont indépendamment un atome d'hydrogène ou un groupe alkyle ou aryle en C_{1 à 10} non substitué ou substitué ;
ou un sel, un complexe de coordination avec des ions de métaux, un ester, un acide ou une base libre, ou un hydrate de celui-ci, ou un dérivé glycolipide dans lequel une fraction lipide pouvant être clivée par une enzyme phospholypase est un substituant d'un résidu de sucre ou du cyclitol.

2. Composé selon la revendication 1, dans lequel le résidu de sucre est un hexose ou un pentose, ou des formes substituées de ceux-ci.

3. Composé selon la revendication 2, dans lequel le résidu de sucre est un hexose choisi dans le groupe constitué par le glucose, le galactose ou le mannose.

4. Composé selon la revendication 2, dans lequel le résidu de sucre est une hexosamine.

5. Composé selon la revendication 4, dans lequel l'hexosamine est la galactosamine ou la glucosamine.

6. Composé selon l'une quelconque des revendications précédentes, qui est choisi dans le groupe constitué par :
RGL1023 *O*-(2'-amino-2'-désoxy-6-phosphate-D-glucopyranosyl)-α(1,6)-D-*myo*-inositol ;
RGL1027 *O*-(2'-amino-2'-désoxy-4'-phosphate-D-glucopyranosyl)-α(1,6)-D-*myo*-inositol ;
RGL1029 *O*-(2'-amino-2'-désoxy-3'-phosphate-D-glucopyranosyl)-α(1,6)-D-*myo*-inositol ;
RGL1134 1'-D-6-*O*-(2'-amino-3'-*O*-benzyl-4'-*O*-phosphate-2'-désoxy-α-D-glucopyranosyl)-3,4,5-tri-*O*-benzyl-*myo*-inositol-1,2-phosphate cyclique ;
RGL1133 1'-D-6-*O*-(2'-amino-3'-*O*-benzyl-4',6'-di-*O-*sulfate-2'-désoxy-α-D-glucopyranosyl)-3,4,5-tri*O*-benzyl-*myo*-inositol-1,2-di-*O*-sulfate ;
RGL1135 1'-D-6-*O*-(2'-amino-3'-*O*-benzyl-4',6'-di-*O-*phosphate cyclique-2'-désoxy-α-D-glucopyranosyl)-3,4,5-tri-*O*-benzyl-*myo*-inositol-1,2-phosphate cyclique ;
RGL1130 1'-D-6-*O*-(2'-amino-4'-O-phosphate-2'-désoxy-α-D-glucopyranosyl)-*myo*-inositol-1,2-phosphate cyclique et 1'-D-6-*O*-(2'-amino-6'-*O*-phosphate-2'-désoxy-α-D-glucopyranosyl)-*myo*-inositol-1,2-phosphate cyclique ;
ou un sel, un complexe de coordination avec des ions de métaux, un ester, un acide ou une base libre, un hydrate de celui-ci, ou un dérivé glycolipide dans lequel une fraction lipide pouvant être clivée par une enzyme phospholipase est un substituant d'un résidu de sucre ou du cyclitol.

7. Composition comprenant un composé selon l'une quelconque des revendications précédentes, en combinaison avec un support pharmaceutiquement acceptable.

8. Composé selon l'une quelconque des revendications 1 à 6, à utiliser dans un procédé de traitement médical.

9. Utilisation d'un composé selon l'une quelconque des revendications 1 à 6, pour la préparation d'un médicament destiné au traitement d'une affection améliorée par l'administration d'un second messager d'inositol phosphoglycane (IPG) ou d'un antagoniste d'IPG.
